(19) Europäisches Patentamt
European Patent Office
Office européen des brevets

(11) Veröffentlichungsnummer: **0 285 000 B1**

(12) # EUROPÄISCHE PATENTSCHRIFT

(45) Veröffentlichungstag der Patentschrift: **28.07.93**

(51) Int. Cl.$^5$: **C07K 5/00**, C07D 471/04, C07C 229/18, C07C 229/24, C07C 229/36, C12Q 1/37, C12Q 1/56, C09B 55/00

(21) Anmeldenummer: **88104715.3**

(22) Anmeldetag: **24.03.88**

(54) **Chromogene Verbindungen, ihre Herstellung und Verwendung als Enzymsubstrate.**

(30) Priorität: **01.04.87 DE 3710937**

(43) Veröffentlichungstag der Anmeldung:
**05.10.88 Patentblatt 88/40**

(45) Bekanntmachung des Hinweises auf die Patenterteilung:
**28.07.93 Patentblatt 93/30**

(84) Benannte Vertragsstaaten:
**AT BE CH DE ES FR GB GR IT LI LU NL SE**

(56) Entgegenhaltungen:
**EP-A- 0 258 784**

**CHEMICAL ABSTRACTS, Band 105, Nr. 2, Juli 1986, Coulumbus, OH (US); H. HENNIG et al., Seite 78, Nr. 7925a&NUM;**

(73) Patentinhaber: **BOEHRINGER MANNHEIM GMBH**
**Sandhofer Strasse 116**
**W-6800 Mannheim 31(DE)**

(72) Erfinder: **Wielinger, Hans, Dr. phil**
**Im Langgewann 7**
**W-6940 Weinheim(DE)**
Erfinder: **Zimmermann, Gerd, Dr. rer. nat.**
**Dornheimer Ring 4**
**W-6800 Mannheim 41(DE)**

EP 0 285 000 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft chromogene Verbindungen, die als Substrate zum Nachweis und zur Bestimmung von Peptidbindungen spaltenden Enzymen geeignet sind.

Peptidbindungen spaltende Enzyme sind solche, die befähigt sind, die Hydrolyse von CONH-Bindungen zu katalysieren. Hierzu werden gerechnet Transpeptidasen, wie z. B. $\gamma$-Glutamyltranspeptidase, Peptidasen, wie z. B. Leucinarylamidase und Proteinasen, wie z. B. Kathepsine, Chymotrypsin, Collagenasen, Elastasen, Enterokinasen, Papain, Trypsin und z. B. auch alle Proteasen, die am Ablauf der Blutgerinnung oder Fibrinolyse beteiligt sind. Insbesondere die Enzyme, die beim Ablauf der Blutgerinnung oder Fibrinolyse beteiligt sind, sowie Trypsin, Chymotrypsin, Leucinarylamidase und $\gamma$-Glutamyltranspeptidase haben in der klinischen Chemie zur Erlangung diagnostischer Aussagen eine große Bedeutung erlangt.

Für den Nachweis und die Bestimmung dieser Enzyme in Körperflüssigkeiten, wie z. B. Blut, Serum, Plasma oder Urin ist es oft wichtig, so schnell wie möglich, genaue Ergebnisse zu erlangen. Da der Anwenderkreis für solche Nachweis- und Bestimmungsverfahren breit ist, ist es notwendig, einfache Verfahren zur Verfügung zu stellen. Diese Verfahren dürfen durch die Probenbeschaffenheit nicht gestört werden. Solche Störungen können z. B. Färbungen bei ikterischen Proben, Trübungen bei lipämischen Proben etc. sein.

Bisher bekannte Substrate für Peptidbindungenspaltende Enzyme sind nach einem Schema aufgebaut, das in Formel I

$R^1$-NH-R (I)

in der

- $R^1$ den Rest einer Aminosäure oder eines Oligopeptids, der gegebenenfalls geschützte Aminogruppen enthält und
- R den Rest einer detektierbaren Verbindung, die mit einer Aminogruppe eine Amidbindung mit dem Carboxylende der Aminosäure oder des Oligopeptids eingegangen ist,

bedeutet, dargestellt ist. Bei der enzymatischen Spaltung solcher Substrate entstehen Carbonsäuren $R^1$-OH-(Ia) (Aminosäuren oder Oligopeptide mit freier Carboxylfunktion) und Amine R-$NH_2$ (Ib).

Als $R^1$ werden am häufigsten einzelne Aminosäuren bis Tetrapeptide verwendet. Es können aber auch größere Oligopeptide eingesetzt werden. Im allgemeinen wird mit dem Substrat der Formel I dem zu bestimmenden Enzym die Aminosäure bzw. die Aminosäuresequenz angeboten, an der es auch im natürlichen Substrat spaltet und die letztendlich für die Spezifität des Substrats für das Enzym hauptsächlich verantwortlich ist. Stellvertretend für die zahlreichen aus der Fachliteratur bekannten Aminosäuren bzw. Aminosäuresequenzen seien für diagnostisch besonders interessante Vertreter einige Beispiele genannt: für Transpeptidasen wie $\gamma$-Glutamyltranspeptidase z. B. der $\gamma$-Glutamylrest (Bergmeyer, "Methods of Enzymatic Analysis", 3rd edition, volume III, 349 - 364, Verlag Chemie, 1983), für Peptidasen wie Leucinaminopeptidase z. B. der Leucinrest (Z. klin. Chem. u. klin. Biochem. 10, 192 (1972)), für Proteinasen wie Trypsin z. B. das N-terminal geschützte Tetrapeptid Ile-Glu-Gly-Arg (Bergmeyer, "Methods of Enzymatic Analysis", 3rd edition, volume V, 124 - 129, Verlag Chemie, 1984) und für Proteasen, die bei den Vorgängen der Blutgerinnung wirken wie Thrombin z. B. die N-terminal geschützten Tripeptide Val-Pro-Arg oder Gly-Pro-Arg usw. (Seminars in Thrombosis und Hemostasis, volume 9, number 3, 179 - 183 (1983)).

Amine R-$NH_2$ stellen detektierbare Verbindungen dar, die durch Einwirkung Peptidbindungen spaltender Enzyme auf Substrate der allgemeinen Formel I freigesetzt werden. In der Regel handelt es sich hierbei um Verbindungen, die durch Bindung an eine Aminosäure oder ein Oligopeptid im Substrat der allgemeinen Formel I eine Verschiebung des Absorptionsmaximums oder des Fluoreszenzemissionsmaximums in einen kurzwelligeren Wellenlängenbereich, verglichen mit der freien Substanz R-$NH_2$, erfahren. Die aus dem Substrat abgespaltene Substanz R-$NH_2$ absorbiert also Licht oder emittiert Fluoreszenz in einem längerwelligen Bereich.

Insgesamt können Substrate der allgemeinen Formel I je nach der Art ihrer bei enzymatischer Hydrolyse freigesetzten detektierbaren Verbindungen in drei Kategorien unterteilt werden.

Die erste Kategorie umfaßt Substrate, die nach Spaltung p-Nitroanilin oder ein Derivat hiervon liefern. Solche Substrate sind z. B. Gegenstand der deutschen Patentanmeldungen DE-A 32 44 030 und DE-A 33 11 287. Die durch Spaltung entstandenen detektierbaren Verbindungen sind gelb und werden zweckmäßigerweise bei 405 nm bis 420 nm gemessen.

Solche Verbindungen haben jedoch den Nachteil, daß sie von zahlreichen aus der Probe herrührenden Einflüssen gestört werden können. Wichtige Störfaktoren sind z. B. die Eigenfarbe von Seren, lipämische Trübungen, erhöhte Bilirubinwerte etc. p-Nitroanilin-oder p-Nitroanilinderivate abspaltende Substrate sind

deshalb nur begrenzt verwendbar. In trockenchemischen Testen, die remissionsphotometrisch gemessen werden, stören die vorgenannten Einflüsse derart, daß solche Substrate dort keinen praktischen Einsatz finden.

Substrate der zweiten Kategorie spalten fluoreszierende Verbindungen, wie z. B. 7-Amino-4-methylcumarin, 4-Trifluormethyl-7-amino-cumarin, 4-Methoxy-β-naphthylamin, β-Naphthylamin, 5-Amino-isophthalsäuredimethylester ab. Weitere fluoreszierende Verbindungen, die aus Substraten für Peptidbindungenspaltende Enzyme freigesetzt werden können, sind in EP-A 0 025 190 genannt.

Ihre Verwendung ist wegen der für Fluoreszenzmessungen nötigen Ausrüstung problematisch. Als Substrate für Routinemessungen haben sie deshalb keine Bedeutung erlangt.

Die dritte Substratkategorie betrifft solche Amide von denen unter enzymatischer Einwirkung Aminophenole, Phenylendiamine oder andere aromatische Amine abgespalten werden, die im Bereich des sichtbaren Lichts nicht ausreichend absorbieren und deshalb zur Farbbildung einer Folgereaktion bedürfen. Zur Farbbildung werden meist oxidative Kupplungsreaktionen herangezogen. Solche Nachweisprinzipien sind z. B. aus DE-A 33 31 588 oder EP-A 0 076 042 bekannt.

Das Erfordernis, die aus Substraten der dritten Kategorie abgespaltenen Verbindungen mit einer oxidierenden Nachfolgereaktion zu koppeln, macht deren Einsatz kompliziert und nur begrenzt möglich. Alle in der Probe enthaltenen Substanzen, die durch Oxidation einen Farbstoff bilden, können solche Nachweisreaktionen stören. Hierzu gehört vor allem das in jedem Plasma oder Serum enthaltene Bilirubin. Es muß bei allen Testen, die nach dem Prinzip der oxidativen Kupplung arbeiten durch eine Vorreaktion ausgeschaltet werden. Außerdem können Oxidationsmittel auch durch Eingriff in der eigentlichen Substratspaltungsreaktion vorangehende Reaktionen stören. So wird z. B. der Ablauf der Gerinnungskaskaden durch Oxidationsmittel beeinflußt.

Es bestand deshalb weiterhin Bedarf an Substraten für Peptidbindungen spaltende Enzyme insbesondere für Transpeptidasen, Peptidasen und Proteinasen, die bei der Spaltungsreaktion direkt einen Farbstoff bilden, dessen Absorptionsmaximum deutlich über 420 nm und dessen maximale Absorptionswellenlänge sich von der maximalen Absorptionswellenlänge des Substrats so unterscheidet, daß ein für die Messung der Enzymaktivität zufriedenstellendes Signal erhalten wird.

Aufgabe der vorliegenden Erfindung war es, solche Substrate bereitzustellen.

Gelöst wird diese Aufgabe durch die neuen Verbindungen der allgemeinen Formel II,

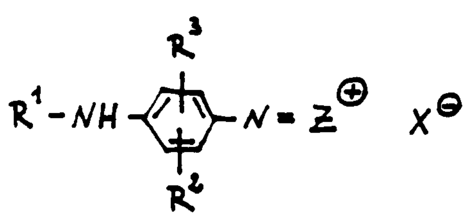

(II)

in der

| | |
|---|---|
| $R^1$ | einen Aminosäurerest oder den Rest eines Oligopeptids darstellt, in dem Aminogruppen gegebenenfalls durch Schutzgruppen substituiert sind, |
| $R^2$, $R^3$, | die gleich oder verschieden sind, Wasserstoff, eine Niederalkyl-, Niederalkoxy-, Carboxy-, Niederalkoxycarbonyl-oder gegebenenfalls durch Niederalkyl substituierte Carboxamidogruppe bedeuten oder falls $R_2$ und $R_3$ benachbart sind, eine -CH=CH-CH=CH-Gruppe darstellen können, |
| Z | ein durchkonjugiertes, ein positiv geladenes Stickstoffatom enthaltendes, bicyclisches heterocyclisches System der Formel III darstellt, |

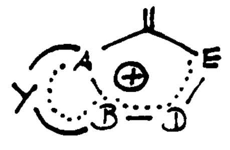

(III)

in der

| | |
|---|---|
| A, B, D und E | ein Kohlenstoff- oder Stickstoffatom bedeuten, wobei sofern sie keine Brückenkopfato- |

me sind,
Kohlenstoffatome gegebenenfalls durch Sauerstoff, eine Niederalkyl-, Niederalkoxy-, Aryl-, Aralkyl- oder Aryloxygruppe und
Stickstoffatome gegebenenfalls durch eine Niederalkyl-, Aralkyl- oder Arylgruppe substituiert sind und

Y eine ungesättigte Kohlenwasserstoffkette mit 3 - 5 Gliedern bedeutet, die gegebenenfalls durch Stickstoff- oder Schwefelatome unterbrochen ist oder solche Heteroatome am Anfang oder Ende der Kette enthält,
wobei Kohlenstoffatome ggfs. durch eine Niederalkyl-, Niederalkoxy-, Aralkyl- , Aryloxy- oder Arylgruppe und/oder Stickstoffatome gegebenenfalls durch eine Niederalkyl-, Aralkyl- oder Arylgruppe
substituiert sind, zwei benachbarte Niederalkylreste gegebenenfalls eine gesättigte Alkylengruppe mit 3 - 5 Kohlenstoffatomen bilden
und insgesamt die Summe der Heteroatome im Bicyclus maximal 3 ist, wiedergegeben werden
und

X ein Anion einer organischen oder anorganischen Säure bedeutet.

Der Aminosäure- oder Oligopeptidrest der erfindungsgemäßen Substrate kann je nach dem zu bestimmenden Enzym gewählt werden. Als Aminosäuren kommen hierbei -, $\beta$- oder -Aminosäuren aus 2 - 15, vorzugsweise 2 - 10 Kohlenstoffatomen in Frage. Handelt es sich hierbei um chirale Aminosäuren, so können diese in der D-oder L-Form oder auch als Racemat vorliegen. Aminosäuren natürlichen Ursprungs, wie sie in Proteinen und Peptidantibiotika vorkommen, sind bevorzugt. Es können aber auch synthetische Aminosäuren, wie z. B. Pipecolinsäure, Cyclohexylalanin, Phenylglycin, -Aminocyclohexylcarbonsäure, Hexahydrotyrosin, Norleucin, oder Ethionin eingesetzt werden. Als Beispiele für Enzyme, die Substrate der allgemeinen Formel II umsetzen, in denen $R^1$ einen Aminosäurerest darstellt, seien genannt -Glutamyltranspeptidase, die Substrate mit einem -Glutamylrest umsetzt, Trypsin, das Substrate mit einem Argininrest spaltet, Aminopeptidase M, die Substrate mit einem aliphatischen Aminosäurerest, wie z. B. Alanin hydrolysiert oder Chymotrypsin, das Substrate mit Phenylalanin erkennt.

Oligopeptidreste in der Bedeutung von $R^1$ enthalten 2 - 10 vorzugsweise 2 - 6, besonders bevorzugt 2 - 4 Aminosäurebausteine. Die Aminosäuren des Oligopeptids können natürlichen und/oder synthetischen Ursprungs sein und entsprechen der vorstehenden Definition für geeignete Aminosäuren in Verbindungen der allgemeinen Formel II.

Je nach zu bestimmendem Enzym sind aus der Literatur (z. B. Seminars in Thrombosis and Hemostasis, Vol. 9, No. 3, S. 179 - 183 (1983), IUPAC Enzyme Nomenclature, 1984, Academic Press, Orlando oder T. E. Barman, "Enzyme Handbook", 1969, Supplement I, 1974, Springer-Verlag, Heidelberg) geeignete Aminosäure- oder Oligopeptidreste bekannt, die als Reste $R^1$ in Substraten der allgemeinen Formel I eingesetzt werden. Sie können analog in erfindungsgemäßen Substraten der allgemeinen Formel II Verwendung finden.

In den Substraten der allgemeinen Formel II können die Terminale und/oder andere eventuell vorhandene Aminogruppen des Aminosäure-oder Oligopeptidrests $R^1$ frei vorliegen. Gegebenenfalls können sie aber auch durch Schutzgruppen substituiert sein.

Als Aminoschutzgruppen sind z. B. Acyl-, Oxycarbonyl-, Thiocarbonyl-, Sulfonyl-, Sulfenyl-, Vinyl-, Cyclohexenyl-, Phosphoryl-oder Carbamoyl-Gruppen zu verstehen. Bevorzugt sind Acyl-, Sulfonyl- und Oxycarbonylgruppen.

Als Acylgruppen kommen Reste aliphatischer oder aromatischer Carbonsäuren, wie z. B. der Formyl-, Acetyl-, Propionyl-, Isobutyryl-, Benzoyl- oder Naphthoylrest in Frage. Bevorzugt sind vor allem der Formyl-, Acetyl- und Benzoylrest.

Bevorzugte Oxycarbonylgruppen sind z. B. die Benzyloxycarbonyl-, die tert.-Butoxycarbonyl-, die Ethoxycarbonyl- und die p-Methoxybenzyloxycarbonylgruppe.

Sulfonylgruppen sind z. B. Reste aliphatischer Sulfonsäuren mit 1 - 6 Kohlenstoffatomen, bevorzugt die Methansulfonylund Ethansulfonylgruppe oder z. B. Reste aromatischer Sulfonsäuren, bevorzugt die Benzolsulfonyl-, Toluolsulfonyl- und Naphthylsulfonylgruppe.

In der Definition der Substituenten $R^2$ und $R^3$ des p-Phenylendiaminteils der Verbindungen der allgemeinen Formel II bedeutet die Niederalkylgruppe einen geraden oder verzweigten Alkylrest mit 1 -6, vorzugsweise 1 - 4 Kohlenstoffatomen. Besonders bevorzugte Reste sind Methyl, Ethyl, iso-Propyl und iso-Butyl.

Eine Niederalkoxygruppe in der Definition von $R^2$ und $R^3$ bedeutet eine gerade oder verzweigte Alkoxygruppe aus 1 - 6, vorzugsweise 1 - 4 Kohlenstoffatomen. Bevorzugte Niederalkoxyreste sind

Methoxy, Ethoxy, Propoxy, iso-Butoxy und tert.-Butoxy.

Die vorangehende Definition für die Niederalkoxygruppe trifft auch für den Niederalkoxyrest in der Niederalkoxycarbonylgruppe der Reste $R^2$ und $R^3$ zu.

Ebenso gilt die oben angegebene Definition der Niederalkylgruppe auch für den Niederalkylsubstituenten einer Carboxamidogruppe in den Resten $R^2$ und $R^3$.

Das Säureanion $X^-$ steht für ein Anion irgendeiner organischen Säure oder Mineralsäure. Bevorzugt sind Anionen starker organischer oder anorganischer Säuren. Besonders bevorzugt sind Acetat-, Trifluoracetat-, Oxalat-, Methansulfonat- oder Sulfat-, chlorid-, Bromid- und Perchlorationen.

Die gestrichelte Linie in Formel III bedeutet hierbei, daß die positive Überschußladung nicht an einem bestimmten Atom des bicyclischen Systems allein lokalisiert ist, sondern durch Mesomerie oder Tautomerie über den cyclisch konjugierten Molekülteil verteilt vorliegt.

Als Brückenkopfatome werden die Atome A und B bezeichnet, die beiden Ringen des bicyclischen Systems gleichzeitig angehören. Sie sind im Rest Z der erfindungsgemäßen Substrate nicht durch andere Atome oder Gruppen substituiert.

Gegebenenfalls kann mindestens ein Kohlenstoffatom in der Definition von D und E, die keine Brückenkopfatome sind durch Sauerstoff substituiert sein und so z. B. eine Carbonylgruppe bilden.

Niederalkyl- bzw. Niederalkoxygruppen, die nicht brückenkopfbildende Kohlenstoff- oder Stickstoffatome in der Definition von D und E substituieren können, sind solche, die aus 1 - 6, vorzugsweise 1 - 4 Kohlenstoffatomen bestehen. Besonders bevorzugte Gruppen sind die Methyl-, Ethyl- und n-Propylgruppe bzw. die Methoxy-, Ethoxy- und n-Propoxygruppe.

Aryl- bzw. Aryloxygruppen, die Kohlenstoff- oder Stickstoffatome in der Definition von D und E substituieren können, sind vorzugsweise die Phenyl- und Naphthyl- bzw. die Phenoxy-und Naphthoxygruppe. Diese können gegebenenfalls ihrerseits noch durch eine oder mehrere 1 - 6, vorzugsweise 1 - 4 Kohlenstoffatome enthaltende Niederalkyl- oder Niederalkoxygruppen oder/und durch ein oder mehrere Halogenatome, wie Fluor, Chlor, Brom oder Jod, vorzugsweise Chlor oder Brom substituiert sein. Besonders bevorzugt sind als Aryl- oder Aryloxysubstituenten die Phenyl- oder Phenoxygruppe.

Aralkylgruppen als mögliche Substituenten von Kohlenstoff-und Stickstoffatomen in der Definition von D und E sind insbesondere die Phenylmethyl- und Naphthylmethylgruppen. Besonders bevorzugt ist die Phenylmethylgruppe.

Unter einer 3 - 5gliedrigen ungesättigten Kohlenstoffwasserkette in der Definition von Y, die gegebenenfalls durch Stickstoff- oder Schwefelatome unterbrochen ist oder solche Heteroatome am Anfang oder Ende der Kette enthält, wird vorzugsweise eine solche Kette verstanden, die durch Anellierung an das andere Ringsystem einen 5- oder 6-Ring bildet. Dieses anellierte Ringsystem enthält eine oder mehrere Doppelbindungen, die zu dem Doppelbindungssystem des anderes Rings konjugiert sind, so daß die positive Überschußladung des heterocyclischen Systems Z durch Mesomerie oder Tautomerie zwischen den beiden Ringen ausgetauscht werden kann. Besonders bevorzugte Ringsysteme für den Y enthaltenden Ring des Bicyclus Z sind das Thiazolium- und Pyridiniumsystem.

Als Niederalkyl-, Niederalkoxy-, Aryloxy-, Aralkyl- und Arylgruppen, die Kohlenstoffatome oder Stickstoffatome der ungesättigten Kette Y substituieren können, kommen die gleichen Gruppen in Frage, wie vorstehend für Kohlenstoff- und Stickstoffatome in der Definition von D und E ausgeführt.

Im Falle, daß innerhalb Y zwei benachbarte Niederalkylreste als Substituenten von Kohlenstoff- und/oder Stickstoffatomen vorliegen, ist es möglich, daß diese eine gesättigte Alkylengruppe mit 3 - 5 Kohlenstoffatomen darstellen und so einen an Y ankondensierten Ring bilden. Bevorzugt sind Alkylengruppen aus 3 oder 4 Kohlenstoffatomen, die einen 5- oder 6-Ring bilden. Besonders bevorzugt ist ein so ankondensierter Cyclopentan- oder Cyclohexanring.

Besonders bevorzugt sind solche Verbindungen der allgemeinen Formel II, in der Z

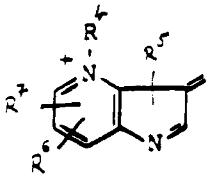

(IV)

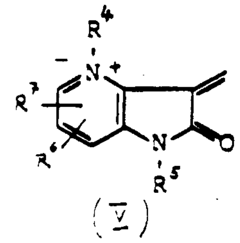

(V)

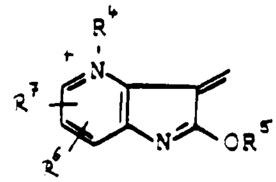

(VI)

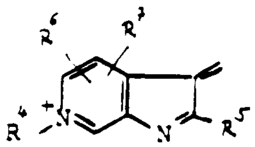

(VII)

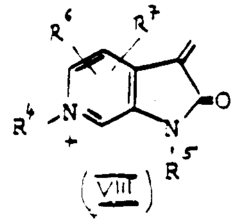

(VIII)

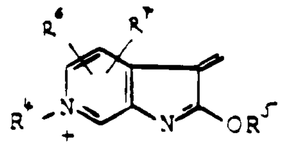

(IX)

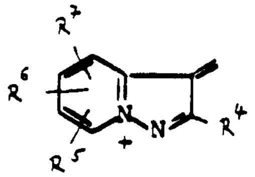

(X)

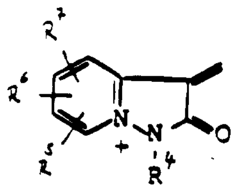

(XI)

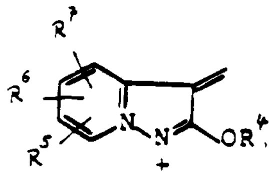

(XII)

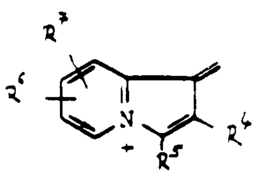

(XIII)

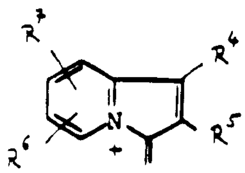

(XIV)

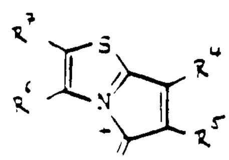

(XV)

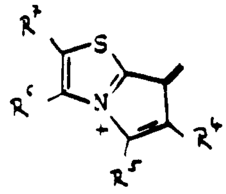

(XVI)

bedeutet, wobei

$R^4$, $R^5$, $R^6$ und $R^7$, die gleich oder verschieden sein können, Wasserstoff, eine Niederalkyl-, Niederalkoxy-, Aralkyl- , Aryloxy- oder Arylgruppe darstellen.

Als Niederalkyl-, Niederalkoxy-, Aralkyl-, Aryloxy- und Arylgruppen in der Definition von $R^4$ - $R^7$ kommen die gleichen Reste in Frage wie vorstehend für die Substituenten in der Definition von D, E und Y der allgemeinen Formel III angegeben.

Ganz besonders bevorzugte Verbindungen der allgemeinen Formel II sind die, die als durchkonjugiertes, positiv geladenes bicyclisches heterocyclisches System Z ein solches der allgemeinen Formel V, VIII, XI, XII, XIV oder XV aufweisen.

Verbindungen der allgemeinen Formel II sind neu.

Aus der Literatur sind Kondensationen von aromatischen oder heteroaromatischen Nitrosoverbindungen mit Phenolen, Anilinen, Methylenbasen bzw. deren Vorstufen, entsprechend methylsubstituierten, quartären Stickstoffheterocyclen wie z. B. 2-oder 4-Picolinjodmethylat, 2-Methyl-1-arylchinoliniumsalze, Pyrazolonen oder Imidazolo-(1, 2a)-pyridin-2-on bekannt (Houben-Weyl Band 10/1, Seite 1079 ff.; Chem. Ber. 90, 2792 (1957); J. Chem. Soc. 914 (1951); F. Hamer in "Cyanine and related dyes", The Chemistry of Heterocyclic compounds, A. Weissberger Ed., Volume 18, Seite 443 ff.; H. Stamm in "Methodicum Chimicum", F. Zymalkowsky Ed., Volume 6, Georg Thieme Verlag, Stuttgart, 1975, Seite 33; Bull. Soc. Chim. Belg. 58, (1949); J. Amer. Chem. Soc. 66, 1805 (1944). Diese Kondensationen laufen in der Hitze und unter basischer Katalyse durch z. B. Piperidin, Natriumhydroxid oder Natriumcarbonat ab. In Einzelfällen, z. B. beim Einsatz quartärer Amoniumsalze kann die Reaktion auch unkatalysiert ablaufen.

Aus Houben-Weyl Band 10/1, Seite 1081 ff. sind auch Kondensationen von 3,5-Diphenyl-2-nitrosopyrrol mit 2,4-Diphenylpyrrol oder von Nitrosobenzol mit 2,4-Diketopiperazin bekannt, die in Gegenwart von Acetanhydrid unter Erhitzen ablaufen. Derartige drastische Reaktionsbedingungen sind jedoch bei Verbindungen, die wie in der vorliegenden Anmeldung Aminosäurebausteine als Strukturelemente enthalten, nicht anwendbar.

Es war deshalb überraschend, daß es möglich ist, Verbindungen der allgemeinen Formel II durch Umsetzung von Nitrosoverbindungen der allgemeinen Formel XVII,

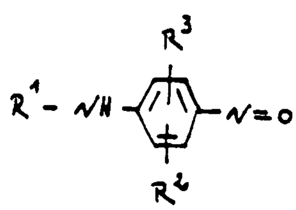

in der

$R^1$ einen Aminosäurerest oder den Rest eines Oligopeptids, in dem Aminogruppen durch Schutzgruppen substituiert sind und

$R^2$, $R^3$ die gleich oder verschieden sind, Wasserstoff, eine Niederalkyl-, Niederalkoxy-, Carboxy-, Niederalkoxycarbonyl- oder eine gegebenenfalls durch Niederalkyl substituierte Carboxamidogruppe oder falls $R^2$ und $R^3$ benachbart sind, eine -CH = CH-CH = CH-Gruppe darstellen,

bedeuten, unter milden Bedingungen und saurer Katalyse mit Verbindungen der allgemeinen Formel XVIII

Z'-H (XVIII)

in der

Z' ein ungeladenes, mindestens ein Stickstoffatom enthaltendes, bicyclisches, heterocyclisches System der allgemeinen Formeln XIXa oder XIXb darstellt

(XIXa) 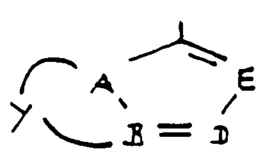 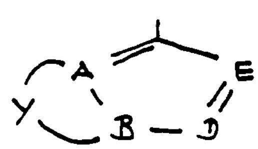 (XIXb)

in denen

A, B, D und E jeweils ein Kohlenstoff- oder Stickstoffatom bedeuten, wobei sofern sie keine Brückenkopfatome sind
Kohlenstoffatome gegebenenfalls durch Sauerstoff, eine Niederalkyl-, Niederalkoxy-, Aryl-, Aralkyl-, Aryloxygruppe und
Stickstoffatome gegebenenfalls durch eine Niederalkyl-, Aralkyl- oder Arylgruppe substituiert sind,

Y eine ungesättigte Kohlenwasserstoffkette mit 3 - 5 Gliedern bedeutet, die gegebenenfalls durch Stickstoff- oder Schwefelatome unterbrochen ist oder solche Heteroatome am Anfang oder Ende der Kette enthält,
wobei Kohlenstoffatome ggfs. durch eine Niederalkyl-, Niederalkoxy-, Aralkyl-, Aryloxy- oder Arylgruppe und/oder Stickstoffatome gegebenenfalls durch eine Niederalkyl-, Aralkyl- oder Arylgruppe bilden,
substituiert sind und zwei benachbarte Niederalkylreste gegebenenfalls eine gesättigte Alkylengruppe mit 3 - 5 Kohlenstoffatomen bilden

insgesamt die Summe der Heteroatome im Bicyclus maximal 3 ist,
die Doppelbindungen der ungesättigten Kette Y in Konjugation zum Doppelbindungssystem des 5-Ringes stehen und/oder wenn A oder B ein Stickstoffatom ist, dieses Enaminstrukturen zu beiden Ringsystemen ausbildet, bedeuten, wiedergegeben wird und gegebenenfalls anschließende vollständige oder teilweise Entfernung von Aminoschutzgruppen zu erhalten.

Die Reaktion zwischen Verbindungen der allgemeinen Formel XVII und XVIII läuft bei Temperaturen zwischen -20 und +50° C ab, bevorzugt bei +10 bis +25° C.

Als saure Katalysatoren sind organische Säuren oder Mineralsäuren geeignet. Aprotische und protische Lösungsmittel können gleichermaßen verwendet werden. Bevorzugte aprotische Lösungsmittel sind z. B. Dimethylformamid oder Methylenchlorid. Bevorzugte protische Lösungsmittel sind Alkohole, wie z. B. Methanol oder Ethanol. Besonders bevorzugt wird jedoch Essigsäure als Reaktionsmedium eingesetzt, wobei diese Säure sowohl Lösungsmittel- als auch Katalysatorfunktion hat.

Umsetzungen von aromatischen Nitrosoverbindungen, insbesondere von solchen der allgemeinen Formel XVII, mit heterocyclischen, bicyclisierten Verbindungen der allgemeinen Formel XVIII unter den vorgenannten Bedingungen waren bisher nicht bekannt.

Besonders bevorzugt werden als Reste Z'

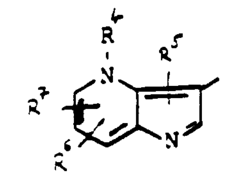
(XX)

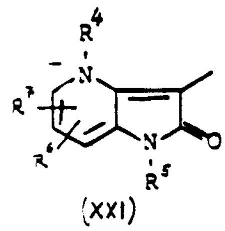
(XXI)

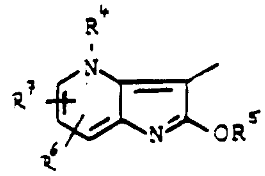
(XXII)

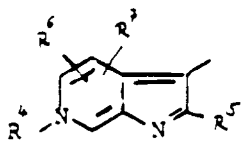
(XXIII)

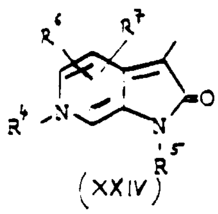
(XXIV)

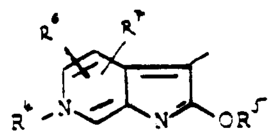
(XXV)

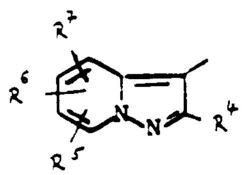
(XXVI)

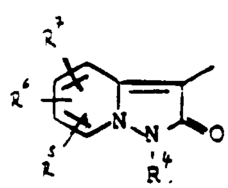
(XXVII)

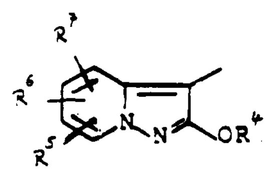
(XXVIII)

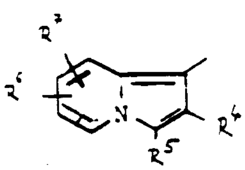
(XXIX)

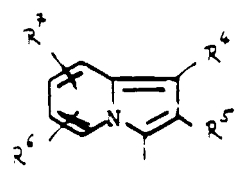
(XXX)

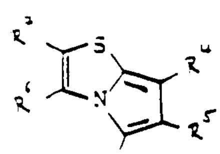
(XXXI)

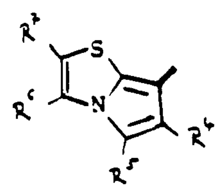
(XXXII)

eingesetzt, wobei diejenigen der Formeln XXI, XXIV, XXVII, XXVIII, XXX und XXXI ganz besonders bevorzugt sind.

Die Bedeutungen der Reste A, B, D, E, Y und $R^1$ - $R^7$ in den Definitionen der allgemeinen Formeln XVII und XIX a/b bis XXXII entsprechen den für die allgemeinen Formeln II bis XVI angegebenen. Dies bezieht die möglichen Aminoschutzgruppen des Aminosäure- oder Oligopeptidrestes $R^1$ mit ein. Derartige Schutzgruppen sowie ihre Abspaltung werden z. B. in Houben-Weyl Band 15/1 beschrieben.

Bicyclen der allgemeinen Formel XVIII oder solcher in denen $Z'$ die in den allgemeinen Formeln XIXa) und b) angegebene Bedeutung hat, sind bekannt oder lassen sich analog bekannter Verbindungen herstellen. Übersichten zur Synthese sind beispielsweise in "Heterocyclic Compounds", A.. Weissberger & E. C. Taylor Eds., Volumes 30 und 46; Adv. Heterocyclic Chem. Volume 33, Seite 185 ff. (1983); Bull. Chem. Soc. Jap. 49, 1980 (1976) und J. Chem. Soc. Perkin I, 1531 (1974) enthalten.

Nitrosoverbindungen der allgemeinen Formel XVII ihrerseits sind neu. Als Zwischenprodukte bei der Herstellung der erfindungsgemäßen Verbindungen der allgemeinen Formel II sind sie ebenfalls Gegenstand der Erfindung.

Sie können hergestellt werden, indem man
Aminosäuren oder Oligopeptide der allgemeinen Formel Ia

$R^1$-OH (Ia)

in der
$R^1$ einen Aminosäurerest oder den Rest eines Oligopeptids darstellt, in dem Aminogruppen durch Schutzgruppen substituiert sind,
mit
a) Phenylendiaminderivaten der allgemeinen Formel XXXIII

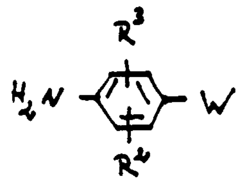

(XXXIII)

in der
$R^2$ und $R^3$, die gleich oder verschieden sind, Wasserstoff, eine Niederalkyl-, Niederalkoxy-, Carboxy-, Niederalkoxycarbonyl- oder eine gegebenenfalls durch Niederalkyl substituierte Carboxamidogruppe oder falls $R^2$ und $R^3$ benachbart sind, eine -CH=CH-CH=CH-Gruppe darstellen, und
W eine durch eine Schutzgruppe substituierte Aminogruppe
bedeuten, umsetzt, anschließend die Aminoschutzgruppe der Gruppe W abspaltet und die hieraus resultierende Verbindung mit freier $NH_2$-Gruppe oxidiert, oder
b) p-Nitranilinen der allgemeinen Formel XXXIV

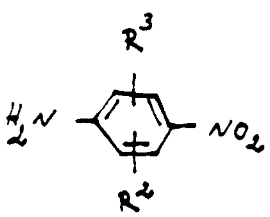

(XXXIV)

in der
$R^2$ und $R^3$ die für Formel XXXIII genannte Bedeutung haben,
umsetzt, die hieraus resultierende Nitroverbindung zum entsprechenden Hydroxylamin reduziert und dann oxidiert.

Die nach Weg b) durch Umsetzungen der Verbindungen der allgemeinen Formeln Ia und XXXIV hergestellten Nitroverbindungen können natürlich auch zu den entsprechenden Aminoverbindungen reduziert werden. Diese sind dann mit den nach Variante a) durch Umsetzung der Verbindungen der allgemeinen Formel Ia und XXXIII sowie anschließende Schutzgruppenentfernung erhaltenen Phenylendiaminderiva-

ten mit einer freien $NH_2$-Gruppe identisch und können wie diese zu Nitrosoderivaten der allgemeinen Formel XVII oxidiert werden.

Die in den Verbindungen der allgemeinen Formel Ia, XXXIII sowie XXXIV aufgeführten Bedeutungen der Reste $R^1$ - $R^3$ entsprechen den in der Formel II angegebenen. Gleiches gilt auch für die Aminoschutzgruppen der geschützten Aminogruppe W, welche denen des Aminosäure- oder Oligopeptidrestes $R^1$ entsprechen. Die Aminoschutzgruppen in $R^1$ und W müssen jedoch innerhalb der nach Variante a) hergestellten Moleküle nicht gleich sein.

Kondensationsreaktionen zwischen Aminosäuren oder Oligopeptiden und Anilinderivaten die denen der allgemeinen Formeln XXXIII und XXXIV analog sind, sind aus dem Stand der Technik bekannt. So sind z. B. ausführliche Zusammenstellungen zu diesem Thema in Houben-Weyl Band 15/1 und Band 15/2 enthalten.

Die Abspaltung der Aminoschutzgruppen aus W erfolgt analog Houben-Weyl Band 15/1.

Die nach Variante a) nach Abspaltung der Aminoschutzgruppe aus W erhaltenen Verbindungen mit freier Aminogruppe können analog zu bekannten Methoden, z. B. Houben-Weyl, Bd. 10/1, S. 1017 f. oxidiert und in Nitrosoverbindungen der allgemeinen Formel XVII überführt werden. Bevorzugt werden hierzu als Oxidationsmittel Caro'sche Säure oder Peroxicarbonsäuren, wie z. B. Peressigsäure eingesetzt.

Aus Houben-Weyl Band 10/1, Seite 1091 ff. sind Verfahren zur Reduktion von Nitroverbindungen zu Hydroxylaminen bekannt. Diese können analog für die Reduktion der nach Variante b) hergestellten Kondensationsprodukte aus Verbindungen der allgemeinen Formel Ia und XXXIV durchgeführt werden.

Die Oxidation so hergestellter Hydroxylaminderivate kann dann mit starken Oxidationsmitteln erfolgen. Besonders bevorzugt sind hierfür Chrom(VI)Oxid oder Natriumperjodat.

Die Überführung von nach Weg b) hergestellten Nitroverbindungen in nach Weg a) hergestellte Verbindungen mit freier $NH_2$-Gruppe kann ebenfalls analog nach bekannten Methoden z. B. durch Reduktion mit Natriumdithionit oder durch Hydrierung über einem geeigneten Katalysator wie z. B. Palladium/Kohle erfolgen.

Die erfindungsgemäßen Substrate der allgemeinen Formel II liegen vorzugsweise in der Salzform vor. Sie werden so hergestellt und zeichnen sich in dieser Form auch durch gute Lagerstabilität aus. Mit Basen lassen sie sich jedoch leicht in die freie, neutrale, ungeladene Form überführen.

Die freie Form kann durch die Formel II'

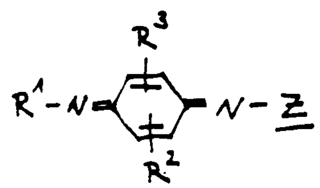

(II')

in der

$R^1$ - $R^3$ die gleiche Bedeutung haben wie in der allgemeinen Formel II und

$\underline{Z}$ ein ungeladenes Mesomeres des durchkonjugierten, positiv geladenen, bicyclischen, heterocyclischen Systems Z darstellt, wiedergegeben werden.

Bei der Synthese von Verbindungen der allgemeinen Formel II entstehen Substanzen, aus denen durch enzymatische Hydrolyse mit Hilfe entsprechender Peptidbindungen spaltender Enzyme Phenylendiaminderivate freigesetzt werden, die bei Wellenlängen von über 420 nm Licht absorbieren. In der Regel sind die enzymatisch freigesetzten Farbstoffe rot bis blau gefärbt.

Gegenüber bisher bekannten Substraten zur Bestimmung Peptidbindungen spaltender Enzyme besitzen die erfindungsgemäßen Verbindungen sehr vorteilhafte Eigenschaften. Sie sind wasserlösliche, stabile Verbindungen, die sowohl in Naß- als auch in Trockentests eingesetzt werden können.

Aufgrund der langwelligen Lichtabsorption der durch enzymatische Hydrolyse freigesetzten Phenylendiaminderivate werden Störungen durch die Eigenfarbe von Seren oder Trübungen vermieden.

Die Messung der Intensität der Lichtabsorption der durch Hydrolyse der Amidbindung freigesetzten Farbstoffe ist wegen des Unterschieds der maximalen Absorptionswellenlängen von erfindungsgemäßen Substraten der allgemeinen Formel II und der nach enzymatischer Spaltung vorliegenden Farbstoffe ($\lambda$ - Shift) mit für die Verfolgung der Hydrolysereaktion ausreichender Genauigkeit möglich.

Da bei der Einwirkung Peptidbindungen spaltender Enzyme auf Verbindungen der allgemeinen Formel II direkt Substanzen gebildet werden, die unmittelbar und ohne weitere Folgereaktion photometrisch bestimmt werden können, ist auch das Problem der Störung der enzymatischen Reaktion durch Zusatz weiterer Reaktionspartner, z. B. von Oxidationsmitteln und Kupplungspartnern für oxidative Kupplungsreaktionen, nicht vorhanden. Die Bestimmung Peptidbindungen spaltender Enzyme kann diesbezüglich störungsfrei durchgeführt werden.

Ein weiterer Gegenstand der Erfindung ist die Verwendung von Verbindungen der allgemeinen Formel II zum Nachweis und zur Bestimmung der Aktivität von entsprechenden Peptidbindungen spaltenden Enzyme. Es hat sich gezeigt, daß die beanspruchten Substrate zur Bestimmung der Aktivität von Peptidbindungen spaltenden Enzymen, insbesondere von Transpeptidasen, Peptidasen und Proteinasen geeignet sind. Beispielsweise können als Transpeptidasen, γ-Glutamyltranspeptidase, als Peptidase Leucinarylamidase oder Aminopeptidase M oder als Proteinasen Kathepsine, Chymotrypsin, Collagenasen, Elastasen, Subtilisin, Enterokinasen, Papain, Trypsin und insbesondere alle Proteasen, die beim Ablauf der Blutgerinnung oder Fibrinolyse beteiligt sind, so nachgewiesen und bestimmt werden. Vor allem zur Bestimmung von γ-Glutamyltranspeptidase, Leucinarylamidase und der Enyzme, die am Ablauf der Blutgerinnung oder Fibrinolyse beteiligt sind sowie von Trypsin und Chymotrypsin haben sich die erfindungsgemäßen Verbindungen als besonders geeignet erwiesen.

Bevorzugt werden zum Nachweis solcher Enzyme Verbindungen der allgemeinen Formel II verwendet, in denen Z die in der allgemeinen Formel III angegebene Bedeutung hat. Besonders bevorzugt werden solche erfindungsgemäßen Verbindungen eingesetzt, in denen Z die Bedeutung der allgemeinen Formeln IV - XVI hat. Hiervon sind die Reste der allgemeinen Formeln V, VIII, XI, XII, XIV und XV als besonders geeignet hervorzuheben.

Aminosäuren oder Aminosäuresequenzen in der Bedeutung des Restes $R^1$ der Verbindungen der allgemeinen Formeln II, die für die Spezifität der erfindungsgemäßen Substrate für bestimmte Enzyme erforderlich sind, sind bekannt. Sie können je nach dem zu bestimmenden Enzym ausgewählt werden.

Die erfindungsgemäßen Verbindungen sind bevorzugt dann einzusetzen, wenn die Aktivität von Enzymen nach kinetischen oder Endpunkts-Verfahren photometrisch bestimmt wird. Hierzu wird eines oder mehrere der beanspruchten Substrate, sowie gegebenenfalls weitere Reagenzien und Hilfsstoffe mit der Probe, die das zu bestimmende Enzym enthält, vermischt. Die Verbindungen der allgemeinen Formel II werden bei Anwesenheit eines entsprechenden Peptidbindungen spaltenden Enzyms hydrolysiert und ein farbbildendes Phenylendiaminderivat freigesetzt. Die hierdurch bewirkte Änderung der Absorption der Reaktionsmischung wird photometrisch gemessen. Durch direkten Vergleich mit einer Standardlösung oder durch indirekten Vergleich mit einer Standardkurve kann dann die Aktivität an zu bestimmendem Enzym in der Probe ermittelt werden.

Die beanspruchten Substrate können z. B. auch bei Blutgerinnungstesten verwendet werden, bei denen entweder die Aktivität von Gerinnungsfaktoren über die Messung ihrer Enzymaktivität bestimmt wird oder bei Globaltesten, die Zeit gemessen wird, die vom Start der Reaktion bis zum Entstehen einer gewissen Enzymaktivität vergeht. Letzteres ist z. B. beim Einphasengerinnungstest nach Quick oder bei der Bestimmung der aktivierten partiellen Thromboplastinzeit der Fall. In beiden Fällen wird die Geschwindingkeit des Entstehens einer bestimmten Menge Farbstoff durch Spaltung des entstandenen Thrombins als Meßgröße herangezogen.

Ein weiterer Gegenstand der Erfindung sind diagnostische Mittel zum Nachweis und zur Bestimmung von Peptidbindungen spaltenden Enzymen. Solche diagnostischen Mittel enthalten neben einem oder mehreren der erfindungsgemäßen Substrate der allgemeinen Formel II ein geeignetes Puffersystem sowie gegebenenfalls weitere geeignete, üblicherweise für solche diagnostische Mittel verwendete Zusatzstoffe, wie beispielsweise Netzmittel, Stabilisatoren usw. Das diagnostische Mittel kann in Form einer Lösung, als Lyophilisat, als Pulvergemisch, Reagenztablette oder auf ein geeignetes Trägermaterial aufgebracht vorliegen.

Das erfindungsgemäße diagnostische Mittel in Form einer Lösung enthält vorzugsweise sämtliche für den enzymatischen Test benötigten Reagenzien. Als Lösungsmittel kommen Wasser oder Gemische von Wasser mit einem wasserlöslichen organischen Lösungsmittel, wie z. B. Methanol, Ethanol, Aceton oder Dimethylformamid in Frage. Aus Haltbarkeitsgründen kann es vorteilhaft sein, die für den Test benötigten Reagenzien auf zwei oder mehr Lösungen zu verteilen, die erst bei der eigentlichen Untersuchung vermischt werden.

Zur Herstellung des diagnostischen Mittels in Form eines Lyophilisats im Gesamtgewicht von jeweils 5 - 20 mg, vorzugsweise etwa 10 mg, wird eine Lösung getrocknet, die sämtliche für den Test benötigten Reagenzien enthält.

Ein diagnostisches Mittel in Form von Pulvermischungen oder Reagenztabletten läßt sich herstellen, indem die Bestandteile des Testes mit üblichen galenischen Zusatzstoffen versetzt und granuliert werden. Zusatzstoffe dieser Art sind z. B. Zuckeralkohole, wie Mannit, Sorbit oder Xylit, oder andere lösliche inerte Verbindungen, wie Polyethylenglykole oder Polyvinypyrrolidon. Die Pulvermischungen oder Reagenztabletten weisen im allgemeinen ein Endgewicht von ungefähr 30 bis 200 mg, vorzugsweise 50 bis 80 mg, auf.

Zur Herstellung des diagnostischen Mittels in Form eines Teststreifens kann ein saug- oder quellfähiger Träger, vorzugsweise Filterpapaier, Cellulose oder Kunstfaservlies mit Lösungen der erforderlichen, üblicherweise zur Herstellung von Teststreifen verwendeten Reagenzien in leicht flüchtigen Lösungsmitteln, wie z. B. Wasser, Methanol, Äthanol oder Aceton imprägniert werden. Dies kann in einem Imprägnierschritt erfolgen. Oft ist es jedoch zweckmäßig, die Imprägnierung in mehreren Schritten durchzuführen, wobei Lösungen eingesetzt werden, die jeweils einen Teil der Bestandteile des diagnostischen Mittels enthalten. So kann beispielsweise in einem ersten Schritt mit einer wässrigen Lösung, die den Puffer und andere wasserlösliche Zusatzstoffe enthält, und dann in einem zweiten Schritt mit einer Lösung, die das Substrat für das Peptidbindungen spaltende Enzym enthält, imprägniert werden. Die fertigen Testpapiere können als solche verwendet werden oder in an sich bekannter Weise an Griffen angeklebt oder vorzugsweise zwischen Kunststoffen und feimaschigen Netzwerken gemäß DE-A 21 18 455 eingesiegelt werden.

Erfindungsgemäße Substrate zur Bestimmung von Peptidbindungen spaltenden Enzymen und insbesondere von Gerinnungsparametern können auch in diagnostischen Mitteln nach EP-A 0 208 218, DE-A 36 10 429 oder in analog zu den in EP-A 0 045 476 beschriebenen Mitteln, auf die hiermit Bezug genommen wird, eingesetzt werden

Die nachfolgenden Beispiele zeigen einige der zahlreichen Verfahrensvarianten, die zur Synthese der erfindungsgemäßen Verbindungen beschritten werden können sowie beispielhaft die Verwendung der neuen Substrate zur Bestimmung von Peptidbindungen spaltenden Enzymen.

In den Beispielen werden die folgenden Abkürzungen verwendet:

| | |
|---|---|
| Ala | = Alanin |
| Arg | = Arginin |
| BOC | = tert. Butoxy-carbonyl |
| Fp | = Schmelzpunkt (in °C) |
| γ-Glu | = γ-Glutaminsäure |
| Gly | = Glycin |
| Nle | = Norleucin |
| Phe | = Phenylalanin |
| Pro | = Prolin |
| Tos | = p-Toluolsulfonyl |
| Hepes | = 4-(2-Hydroyxyethyl)-1-piperazin-ethansulfonsäure |
| Tris | = Tris-(hydroxymethyl)-aminomethan |

Die angegebenen $R_f$-Werte wurden durch Dünnschichtchromatographie auf Kieselgelplatten ermittelt.

**Beispiel 1:**

Herstellung von 2H-1,4-Dihydro-4-methyl-2-oxo-3-(4-tosylglycyl-L-prolyl-L-arginyl-amino)-phenylimino)-pyrrolo-[3,2-b]-pyridinium-Sulfat

a) 5,7 g des Tripelsalzes der angenäherten Formel 2 $KHSO_5$ x $K_2SO_4$ x $KHSO_4$ (Caroat®, Firma Degussa, Hanau, Deutschland) werden in 150 ml Wasser gelöst und die Lösung durch Zugabe von Kaliumcarbonat auf pH 7 gestellt. Diese Lösung tropft man zu einer Lösung von 3,94 g Tosyl-glycyl-L-propyl-L-arginin-(4-aminoanilid) in 210 ml Wasser, rührt die Mischung noch ca. 5 Minuten und filtriert den Niederschlag ab. Zur Reinigung wird der Niederschlag in Eisessig gelöst. Von etwas ungelöstem braunem Niederschlag wird abfiltriert und das Filtrat unter Rühren in Ether getropft. Der ausgefallene, gelbbraune Niederschlag wird abfiltriert. Man erhält 3 g (81 %) Tosyl-glycyl-L-prolyl-L-arginyl-(4-nitrosoanilid)-Halbsulfat.

$R_f$ = 0,65, n-Butanol-Eisessig-Wasser 2:1:1.

b) Analog dem entsprechenden Ethylester, dessen Synthese in J. Chem.Soc. Perkin I, 1531 (1974) beschrieben ist, stellt man 2,4-Dihydro-2-hydroxy-pyrrolo-(3,2-b)-pyridin-3-carbonsäuremethylester her. Fp 270° C (Zers.). 6,27 g dieses Methylesters werden in 100 ml Dimethylformamid suspendiert und mit 2,1 ml Methyljodid versetzt. Unter Rühren gibt man portionsweise 1,43 g 55 gewichtsprozentiges Natriumhydrid in Mineralöl zu und rührt das Gemisch anschließend noch 2 Stunden bei Raumtemperatur. Das Reaktionsgemisch wird eingedampft und an Kieselgel mit Aceton/Methanol (90:10), Methanol und Methanol/$H_2O$ (80:20) chromatographiert. Man erhält 6 g (88 %) 4H-2-Hydroxy-4-methyl-pyrrolo-(3,2-b)-pyridin-3-carbonsäuremethylester vom Fp. 175°C.

0,5 g des oben erhaltenen Esters werden mit 30 ml conc. HCl 2 Stunden unter Rückfluß gekocht. Das Reaktionsgemisch wird eingedampft, der Rückstand in Methanol gelöst und das Produkt durch Zugabe von Ether ausgefällt. Man erhält 0,3 g (84 %) 2H-1,4-Dihydro-4-methyl-pyrrolo-(3,2-b)-pyridin-2-on vom

Fp 220° C.

c) 0,684 g der in Beispiel 1a) erhaltenen Nitrosoverbindung werden in 7 ml Eisessig gelöst und unter Rühren tropfenweise mit einer Lösung von 0,148 g 2H-1,4-Dihydro-4-methyl-pyrrolo-(3,2-b)-pyridin-2-on aus Beispiel 1b) in 6 ml Dimethylformamid versetzt. Die rote Lösung wird 8 Stunden bei Raumtemperatur gerührt, über Nacht im Kühlschrank aufbewahrt, eingeengt und der Rückstand in einer Kieselgelsäule mit n-Butanol/Eisessig/Wasser (2:1:1) chromatographiert. Die produkthaltigen Fraktionen werden vereinigt und eingeengt. Der Rückstand wird mit Ether digeriert und abgesaugt. Man erhält 0,17 g einer roten Substanz folgender Struktur:

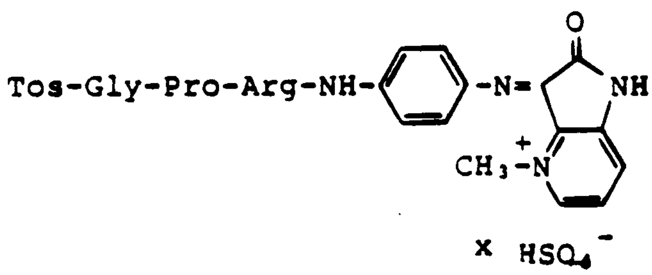

$R_f$ = 0,18 (n-Butanol-Eisessig-Wasser (2:1:1), Fp 167 - 170 °C.

**Beispiel 2:**

Analog Beispiel 1 erhält man durch Umsetzung von Tosyl-glycyl-L-prolyl-L-arginin-4-nitroso-anilid und den in der Tabelle aufgeführten heterocyclischen Verbindungen die folgenden chromogenen Peptidhydrolasesubstrate.

Tabelle 1

R = Tos-Gly-Pro-Arg-NH- 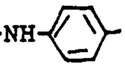

| Beispiel Nr. | Struktur des chromogenen Substrats | $R_f$ [1] Wert | Fp. | Struktur des Ausgangsheterocyclus |
|---|---|---|---|---|
| 2a) | 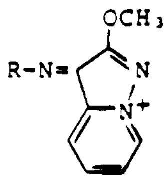 $HSO_4^-$ | 0.31 | 190° C (Zers.) | 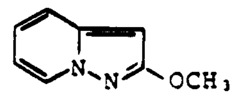 |
| 2b) | 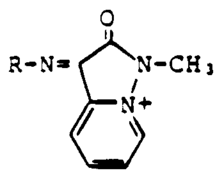 $HSO_4^-$ | 0.22 | 163° C (Zers.) | 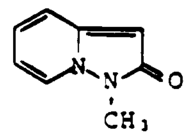 |
| 2c) | 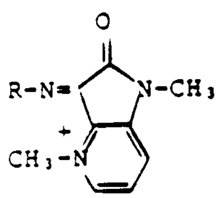 $HSO_4^-$ | 0.11 | 181° C (Zers.) | 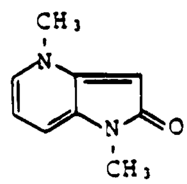 |
| 2d) | 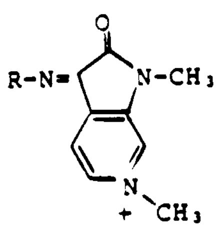 $HSO_4^-$ | 0.33 | 47° C (Zers.) | 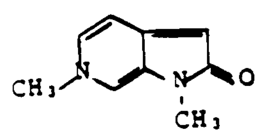 |
| 2e) | 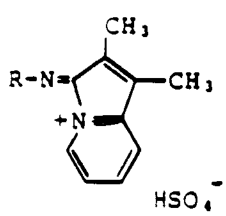 $HSO_4^-$ | 0.18 | 220° C (Zers.) | 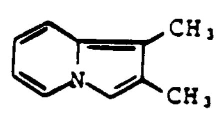 |

1) Kieselgel, Laufmittel n-Butanol/Eisessig/Wasser = 2:1:1

Tab. 1 Fortsetzung

| Beispiel Nr. | Struktur des chromogenen Substrats | $R_f$ [1] Wert | Fp. | Struktur des Ausgangsheterocyclus |
|---|---|---|---|---|
| 2f) | 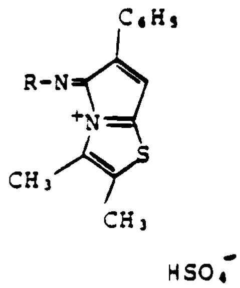 | 0.59 | 165° C (Zers.) | 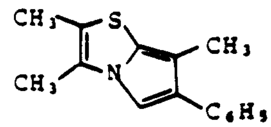 |
| 2g) | 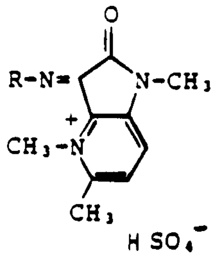 | 0.15 | 135° C | 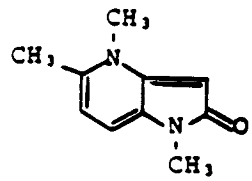 |
| 2h) | 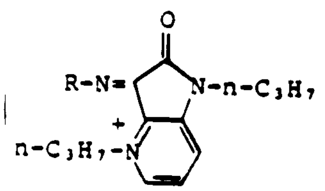 | 0.44 | 168° C (Zers.) | 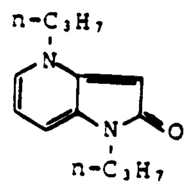 |
| 2i) | 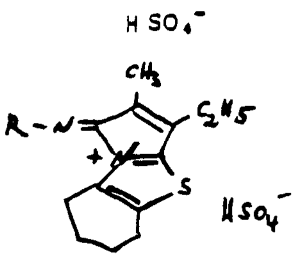 | 0.50 | 170° C (Zers.) | 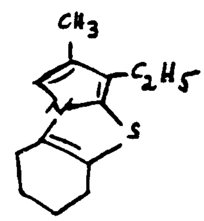 |
| 2 k) | 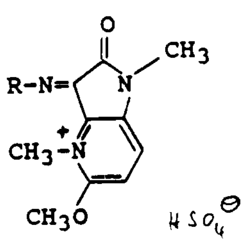 | 0.52 | 171° C (Zers.) | 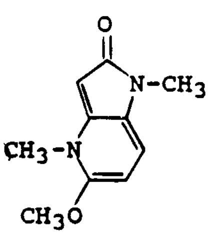 |

1) Kieselgel, Laufmittel n-Butanol/Eisessig/Wasser 2:1:1

Herstellung des Ausgangsheterocyclus für Beispiel 2c)

5 g 2,3-Dihydro-2-hydroxy-pyrrolo-(3.2-b)-pyridin-carbonsäureethylester werden in 180 ml Dimethylformamid suspendiert. Man setzt 10 ml Methyljodid und anschließend protionsweise 4 g Natriumhydrid (55

%ig) hinzu. Das Reaktionsgemisch wird 3 Stunden bei Raumtemperatur gerührt und eingedampft. Der Rückstand wird in Wasser gelöst. Die Lösung wird mehrfach mit Methylenchlorid extrahiert.Die vereinigten organischen Phasen werden getrocknet und eingedampft. Der Rückstand wird in wenig heißem Aceton gelöst und das Produkt mit Ether ausgefällt. Man erhält 3,2 g der entsprechenden N,N′-Dimethylverbindung, vom Fp 145 - 147 °C, die mit 110 ml conc. Salzsäure 24 Stunden unter Rückfluß gekocht wird. Das Reaktionsgemisch wird zur Trockene eingedampft, in Wasser gelöst und die Lösung durch Zugabe von $Na_2CO_3$ alkalisch gestellt. Man extrahiert mehrfach mit Methylenchlorid. Die organischen Phasen werden vereinigt und eingedampft. Man erhält 1,9 g (90 %) 2H-1,4-Dihydro-1,4-dimethyl-pyrrolo-(3,2-b)-pyridin-2-on vom Fp 144 °C.

Herstellung des Ausgangsheterocyclus für Beispiel 2d)

Analog zur Herstellung des Ausgangsheterocyclus für Beispiel 2c) erhält man aus 2,3-Dihydro-2-hydroxy-pyrrolo-(2,3-c)-pyridin-3-carbonsäureethylester die N,N′-Dimethylverbindung 2H-1,6-Dihydro-1,6-dimethyl-pyrrolo-(2,3-c)-pyridin-2-on. Das Rohprodukt wird an Kieselgel mit Methylenchlorid/Methanol 9:1 chromatographiert. Man erhält eine gelbe amorphe Substanz.
$R_f$ = 0,46 (Methylenchlorid/Methanol 9:1).

Herstellung des Ausgangsheterocyclus für Beispiel 2g)

Analog zur Herstellung des Ausgangsheterocylcus für Beispiel 2c) erhält man aus 2,3-Dihydro-5-methyl-2-hydroxy-pyrrolo-(3,2-b)-pyridin-3-carbonsäureethylester (Fp 252 ° C, hergestellt analog der 5-H-Verbindung, J. Chem. Soc. Perkin I, 1521 (1974)) die N,N'-Dimethylverbindung 2H-1,4-Dihydro-1,4,5-trimethyl-pyrrolo-(3,2-b)-pyridin-2-on vom Fp 195 - 200 °C.

Herstellung des Ausgangsheterocyclus für Beispiel 2h)

Analog zur Herstellung des Ausgangsheterocyclus für Beispiel 2c) erhält man durch Alkylierung mit n-Propyljodid mit anschließender Verseifung und Decarboxylierung des Carbonsäureethylesters 2H-1,4-Dihydro-1,4-di-propyl-pyrrolo-(2,3-b)-pyridin-2-on als öliges Produkt.
$R_f$ = 0,4 (Essigsäureethylester/Ethanol 9:1).

Herstellung des Ausgangsheterocyclus für Beispiel 2 i)

10,5 g Buttersäureamid und 5,4 g Phosphorpentasulfid werden in 8 ml Toluol suspendiert und zunächst unter Kühlung, dann bei ca. 75 - 80 °C mit einer Lösung von 21,5 g 2-Bromcyclohexanon in 10 ml Toluol versetzt. Man erhitzt anschließend 15 Minuten unter Rückfluß, läßt abkühlen und stellt den pH der Reaktionsmischung mit 2N Natronlauge auf 8 - 9. Das Produkt wird mit Essigsäureethylester extrahiert. Zur Reinigung wird das Produkt destilliert (Kp. 77- 84 °C, 0,1 mm Hg) und mit Ligroin/Essigsäureethylester (10:1) über Kieselgel filtriert. Man erhält 3.1 g 2-n-Propyl-4,5,6,7-tetrahydro-benzo-1,3-thiazol ($R_f$ = 0.35, Ligroin/Essigsäureethylester 90:10). 2,7 g dieser Verbindung werden in 25 ml Aceton gelöst, mit 3,06 g Bromaceton versetzt und 14 Stunden unter Rückfluß erhitzt. Das Aceton wird abdestilliert und der Rückstand mit 335 ml Ethanol und 8,7 g Natriumethylat versetzt. Das Gemisch wird 1 Stunde unter Rückfluß erhitzt und auf ca. 50 ml eingeengt. Man gießt die Lösung in 500 ml Wasser und extrahiert mit Essigsäureethylester. Das Rohprodukt wird mit Ligroin/Essigsäureethylester (98:2) über Kieselgel chromatographiert. Man erhält 1 g des gewünschten Pyrrolo-[2,1-b]-thiazols der folgenden Struktur (m/e = 219):

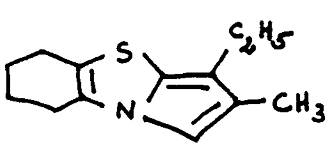

Herstellung des Ausgangsheterocyclus für Beispiel 2 k)

Zu einer Suspension von K-tert. butylat (hergestellt aus 12.48 g Kalium und 600 ml t-Butanol) gibt man bei ca. 80 °C 48.5 ml Malonsäurediethylester. Zu der weißen Suspension tropft man eine heiße Lösung von

30.2 g 2-Chlor-6-methoxy-3-nitropyridin in 200 ml t-Butanol zu. Das Reaktionsgemisch wird 17 Stunden unter Rückfluß erhitzt. Man dampft zur Trockne ein, suspendiert den Rückstand in Wasser, neutralisiert mit 2 N Salzsäure und extrahiert mehrfach mit Diethylether. Die getrocknete Etherphase wird eingeengt und der Rückstand durch Chromatographie an Kieselgel mit Essigsäureethylester/Ligroin (1:9) gereinigt. Man erhält 40.4 g (81 %) 2-(6-Methoxy-3-nitro)-pyridyl-malonsäurediethylester vom Fp 84-85 °C.

20 g der so erhaltenen Substanz werden in 500 ml Ethanol/Methylenchlorid (4:1) gelöst und über einem Palladium auf Kohle Katalysator hydriert. Der Katalysator wird abfiltriert, das Filtrat auf ca. 250 ml eingeengt und mit 25 ml Eisessig versetzt. Das Gemisch wird 4 Stunden unter Rückfluß erhitzt und im Kühlschrank über Nacht stehengelassen. Der ausgefallene Niederschlag wird abfiltriert. Aus der Mutterlauge erhält man durch Einengen auf ca. 100 ml und Versetzen mit ca. 500 ml Diethylether eine weitere Menge Produkt. Insgesamt beträgt die Ausbeute an 1,4-Dihydro-2-hydroxy-5-methoxy-pyrrolo-[3,2-b]-pyridin-3-carbonsäureethylester 10.8 g (72 %) vom Fp 184 - 186 °C.

Analog zur Herstellung des Ausgangsheterocyclus für Beispiel 2 c erhält man aus der oben beschriebenen Verbindung 2H-1,4-Dihydro-1,4-dimethyl-5-methoxy-pyrrolo-[3,2-b]-pyridin-2-on vom Fp 156 - 159 °C.

**Beispiel 3:**

Herstellung von 2H-1,4-Dihydro-4-benzyl-2-oxo-3-(4-(N-methoxy-carbonyl-D-norleucyl-glycyl-L-arginylamido)-phenylimino)-pyrrolo-[3,2-b]-pyridinium-Sulfat

0,95 g N-Methoxycarbonyl-D-norleucyl-glycyl-L-arginin-4-nitranilid-acetat werden in einem Gemisch aus 20 ml Ethanol, 10 ml Wasser und 1 Tropfen Eisessig gelöst und über Palladium/Kohle hydriert. Das Gemisch wird eingedampft, in 300 ml Wasser gelöst und gefriergetrocknet. Man erhält 0,89 g (100 %) N-Methoxycarbonyl-D-norleucyl-glycyl-L-arginin-(4-amino-anilid)-acetat von Fp 108° C.

Diese Verbindung wird analog Beispiel 1a) in die Nitrosoverbindung überführt und analog Beispiel 1c) mit 2H-1,4-Dihydro-4-benzyl-pyrrolo-(3,2-b)-pyridin-2-on zum chromogenen Peptidhydrolasesubstrat der folgenden Struktur umgesetzt.

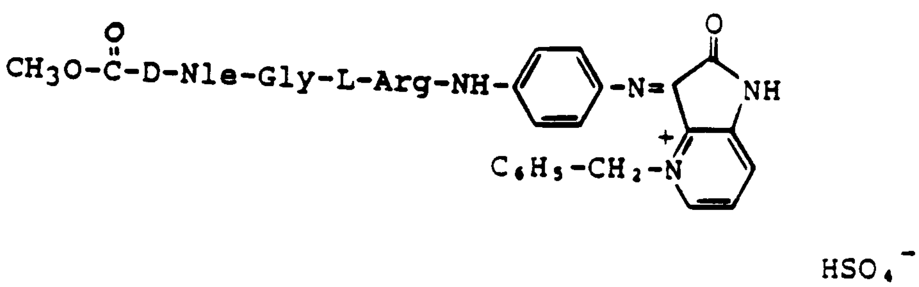

Fp 95 - 97° C,
$R_f$ = 0,08 (Essigsäureethylester/Aceton/Eisessig/Wasser 50:25:12,5:12,5).

**Beispiel 4:**

Herstellung von 2H-1,4-Dihydro-1,4-dimethyl-2-oxo-3-(4-(L-γ-glutamylamido)-phenylimino)-pyrrolo-[3,2-b]-pyridinium-bis-Trifluoracetat

a) 2,85 L-γ-Glu-p-nitranilid Hydrat werden in 25 ml Dioxan suspendiert und mit 1,15 g 1,1,3,3-Tetramethylguanidin und 25 ml Wasser versetzt. Hierzu gibt man eine Lösung von 2,4g Di-tert. butyldicarbonat in 4 ml Dioxan und rührt die Mischung 4 Stunden bei Raumtemperatur. Das Reaktionsgemisch wird mit Wasser versetzt und ausgeäthert. Die wässrige Phase wird durch Zugabe von 2N Salzsäure auf pH 3 gestellt und mit Essigsäureethylester extrahiert. Die organische Phase wird getrocknet und eingedampft. Der Rückstand wird durch Zugabe von Ligroin kristallin. Man erhält 3,3 g BOC-L-γ-Glu-p-nitranilid, das, in einer Mischung von 150 ml Ethanol und 50 ml Wasser gelöst, über Palladium/Kohle hydriert wird. Nach dem Abfiltrieren des Katalysators und Eindampfen des Filtrats verbleiben 2,99 g (88 %) BOC-L-γ-Glu-(4-aminoanilid).

b) 2,47 g der in Beispiel 4a) erhaltenen Verbindung werden in 70 ml 2,5 proz. $NaHCO_3$-Lösung gelöst und unter Rühren mit einer Lösung von 500 mg des Tripelsalzes $2KHSO_5$ x $KHSO_4$ x $K_2SO_4$ (Caroat®,

Firma Degussa, Hanau, Deutschland) in 2 ml Wasser versetzt. Nach zweistündigem Rühren bei Raumtemperatur wird mit Eisessig angesäuert und mit Essigsäureethylester extrahiert. die organische Phase wird getrocknet und eingedampft. Man erhält 1,95 g (76 %) BOC-L-γ-Glu-(4-nitroso-anilid).
$R_f$ = 0,8 (Aceton/Methylenchlorid/Eisessig 50:45:5).
c) 1,9 g der in Beispiel 4b) erhaltenen Nitrosoverbindung werden in 70 ml Eisessig gelöst und mit 0,8 g 2H-1,4-Dihydro-1,4-dimethyl-pyrrolo-(3,2-b)-pyridin-2-on versetzt. Das Gemisch wird 2 Stunden bei Raumtemperatur gerührt und das Produkt durch Zugabe von Äther ausgefällt und abfiltriert. Man reinigt das Rohprodukt durch Chromatographie an Kieselgel mit Methanol und Methanol/Wasser (90:10) als Eluens. Die produkthaltigen Fraktionen werden vereinigt und eingedampft. Man erhält 0,74 g (25 %) einer Verbindung der folgenden Struktur.

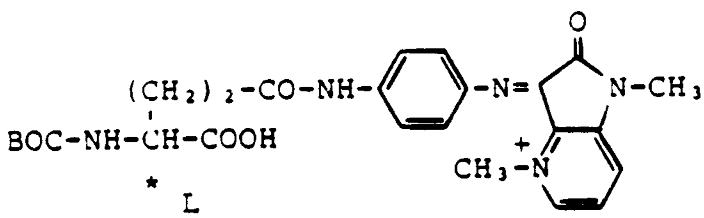

x $CH_3COOH$

$R_f$ = 0,15 ($CH_3OH/H_2O$ 9:1).
d) 0,64 g der in Beispiel 4c) erhaltenen Verbindung werden in
10 ml Trifluoressigsäure gelöst und die Lösung nach 30 Minuten zur Trockene eingedampft. Der Rückstand wird aus $CH_3OH$/Diethylether umkristallisiert. Man erhält 0,7 g (100 %) einer Verbindung der folgenden Struktur.

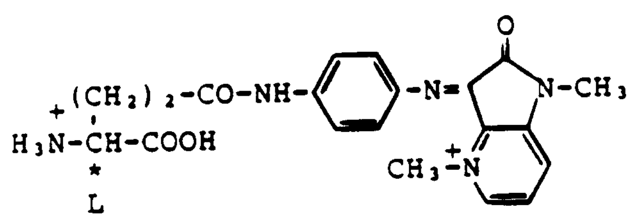

x 2 $CF_3COO^-$

Fp 167 °C, $R_f$ = 0,1 (n-Butanol/Eisessig/Wasser 2:1:1).

**Beispiel 5:**

Herstellung von 2H-1,4-Dihydro-1,4-dimethyl-2-oxo-3-(4-N-succinyl-L-phenylalanyl-amido)-phenylimino)-pyrrolo-[3,2-b]-pyridinium-Halbsulfat

10 g N-Succinyl-L-phenylalanin-p-nitranilid werden in einer Mischung aus 300 ml Ethanol und 100 ml Wasser über Palladium/Kohle hydriert. Man erhält 9,2 g (99 %) N-Succinyl-L-phenylalanin-(4-amino-anilid) vom Fp 167 - 168 °C (Zers.). Diese Verbindung wird analog Beispiel 4c) in das chromogene Substrat folgender Struktur umgewandelt.

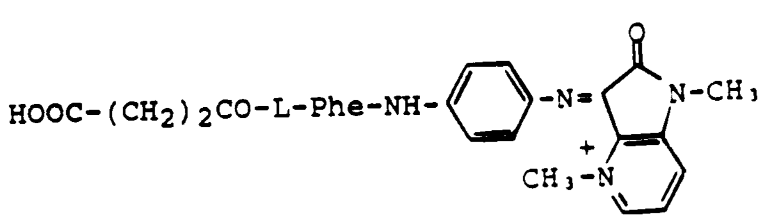

1/2 $SO_4^{2-}$

Fp 158 - 162 ° (Z), $R_f$ = 0,2 (Essigsäureethylester/Aceton/Eisessig/Wasser 50:25:12,5:12,5).

**Beispiel 6:**

Herstellung von 1,2-Dimethyl-5-ethyl-3-(4-(N-succinyl-L phenylalanylamido)-phenylimino)-indolizinium-Halbsulfat

Die in Beispiel 5 erhaltene Nitroso-Verbindung wird analog Beispiel 4c) mit 1,2-Dimethyl-5-ethylindolizin umgesetzt, wobei die Nitroso-Verbindung in 1,4-fachem Überschuß eingesetzt und die Reaktionszeit auf 15 Minuten begrenzt wird. Man erhält eine Verbindung der folgenden Struktur

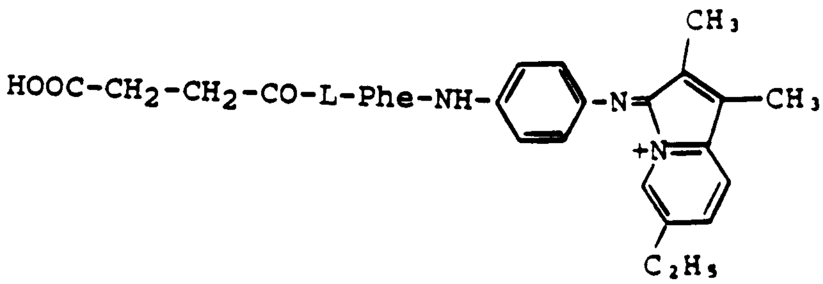

x $CH_3COO^-$

die durch Chromatographie an Kieselgel mit Methanol/$H_2O$ (90:10) als Elutionsmittel gereinigt wird.
Fp 125 - 130 °C (Z)
$R_f$ = 0,25 Essigsäureethylester/Aceton/Eisessig/Wasser (50:25:12,5:12,5)

**Beispiel 7:**

Herstellung von 1,2-Dimethyl-3-(4-N-tert.butoxycarbonyl-L-alanylamido)-2-methoxy-phenylimino)-indolizinium-acetat

a) 50 g 2-Methoxy-4-nitroanilin werden in 820 ml Essigsäureethylester gelöst und mit 70,45 g Chlorameisensäurebenzylester versetzt. Die Mischung wird 5 Stunden unter Rückfluß erhitzt, abgekühlt und die ausgefallenen Kristalle abfiltriert. Man erhält 54,6 g (61 %) N-Benzyloxycarbonyl-2-methoxy-4-nitroanilin, das in einer Mischung von 500 ml Tetrahydrofuran und 500 ml Wasser gelöst wird und unter Kühlen protionsweise mit 210 g Natriumdithionit versetzt wird. nach der vollständigen Reduktion wird die Mischung mit 500 ml Wasser verdünnt und mit Essigsäureethylester extrahiert.
Der Extrakt wird mit Natriumsulfat und Molekularsieb getrocknet und eingeengt. Man erhält 26,8 g (59 %) 4-Benzyloxy-carbonyl-amido-3-methoxy-anilin vom Fp 65 - 68° C.
b) 1,89 g BOC-L-alanin werden in 40 ml Tetrahydrofuran gelöst und mit 1,1 ml N-Methylmorpholin versetzt. Bei -15° C tropft man dazu 1,31 ml Chlorameisensäure-isobutylester. Nach 15 Minuten wird bei - 15° C eine Lösung von 2,75 g der in Beispiel 7a) erhaltenen Verbindung in 10 ml Tetrahydrofuran zugesetzt. Man rührt die Mischung 15 Minuten bei -15° C, 1 Stunde bei -10° C und läßt schließlich auf Raumtemperatur aufwärmen. Die eingedampfte Reaktionsmischung wird in Essigsäureethylester aufgenommen und mit 5 proz. $NaHCO_3$-Lösung durchgeschüttelt. Man trocknet die organische Phase und dampft ein. Das Rohprodukt wird aus Ethanol umkristallisiert. Man erhält 2,58 g (58 %) BOC-L-Ala-(4-benzyloxy-carboxamido-3-methoxy-anilid) vom Fp 144 - 145 °C.
c) 2,55 g der in Beispiel 7b) erhaltenen Verbindung werden in 70 ml Dimethylformamid gelöst und über Palladium/Kohle hydriert. Der Katalysator wird abfiltriert und das Filtrat eingedampft. Man erhält 1,72 g (97 %) BOC-L-Ala-(4-amino-3-methoxy-anilid) vom Fp 115 - 129 °C.
d) Analog Beispiel 4 b) wird die oben erhaltene Verbindung zur Nitrosoverbindung oxidiert und analog Beispiel 4 c) mit 1,2-Dimethylindolizin umgesetzt, wobei die Nitrosoverbindung in 1,4fachem Überschuß eingesetzt und die Reaktionszeit auf 15 Minuten beschränkt wird. Man erhält eine Verbindung der folgenden Struktur

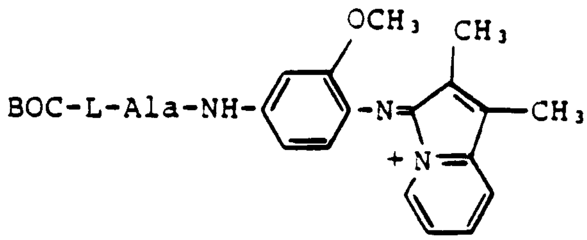

x $CH_3COO^-$

die durch Chromatographie an Kieselgel mit Essigsäureethylester/Aceton/Eisessig/Wasser (50:25:12,5:12,5) als Elutionsmittel gereinigt wird.
$R_f$ = 0,28 (Essigsäureethylester:Aceton:Eisessig:Wasser 50:25:12,5:12,5)
$R_f$ = 0,58 (n-Butanol/Eisessig/Wasser 2:1:1)

**Beispiel 8:**

Herstellung von 1,2-Dimethyl-3-(4-(L-alanylamido)-2-methoxy-phenylimino)-indolizinium-trifluoracetat

Die Verbindung aus Beispiel 7 wird analog Beispiel 4d) zur Abspaltung der BOC-Gruppe mit Trifluoressigsäure behandelt. Man erhält eine Verbindung der folgenden Struktur

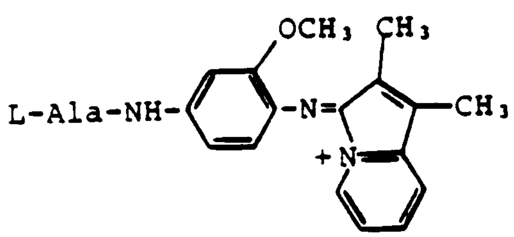

x $CF_3COO^-$
x $CF_3COOH$

Fp 78 °C (Z), $R_f$ = 0,15 (n-Butanol/Eisessig/Wasser 2:1:1).

**Beispiel 9:**

Herstellung von 2H-1,4-Dihydro-1,4-dimethyl-2-oxo-3-(4-(N-tert.butoxy-L-alanyl-L-alanylamido)-2-methyl-phenylimino)-pyrrolo-[3,2-b]-pyridinium-acetat

a) Analog Beispiel 7a) stellt man aus 50 g 2-Methyl-4-nitroanilin 81,3 g (88 %) N-Benzyloxycarbonyl-2-methyl-4-nitroanilin her, das dann mit Natriumdithionit reduziert wird und 44 g 4-Benzyloxycarbonylamido-3-methyl-anilin vom Fp 60 -62 ° C liefert.
Analog Beispiel 7b) werden 2,6 g BOC-L-Ala-Ala in Dimethylformamid mit dem oben erhaltenen p-Phenylendiaminderivat umgesetzt. Ausbeute 1,35 g (27 %)
Fp 197 - 199 ° C.
Das erhaltene Produkt wird über Palladium/Kohle hydriert. Man erhält 1,19 g BOC-L-Ala-Ala-(4-amino-3-methylanilid) vom Fp 135 °C.
b) Die in Beispiel 9a) erhaltene Verbindung wird analog Beispiel 4b) zur Nitrosoverbindung oxidiert und gemäß Beispiel 4c) mit 4H-1,4-Dihydro-1,4-dimethyl-2-oxo-pyrrolo-(3,2-b)-pyridin-2-on umgesetzt. Man erhält eine Verbindung der folgenden Struktur

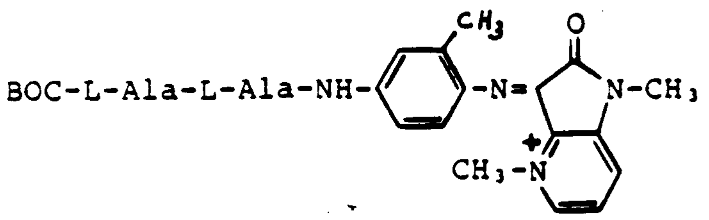

x $CH_3COO^-$

$R_f$ = 0,2 (Essigsäureethylester/Aceton/Eisessig/Wasser 50:25:12,5:12,5).

**Beispiel 10:**

Herstellung von 2H-1,4-Dihydro-1,4-dimethyl-2-oxo-3-(4-L-alanyl-L-alanylamido)-2-methyl-phenylimino)-pyrrolo-[3,2-b]-pyridinium-bis-Trifluoracetat

Die Verbindung aus Beispiel 9a) wird analog Beispiel 4d) zur Abspaltung der BOC-Gruppe mit Trifluoressigsäure behandelt. Man erhält eine Verbindung der folgenden Struktur

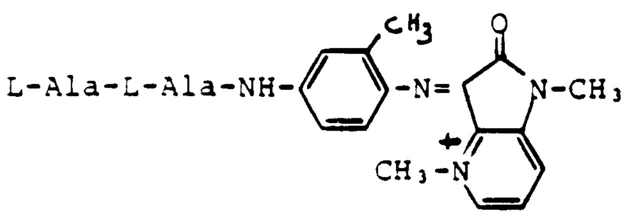

x 2 $CF_3COO^-$

Fp 127 - 132 °C (Zers.)
$R_f$ = 0.10 (n-Butanol/Eisessig/Wasser 2:1:1)

**Beispiel 11:**

Herstellung von 3-(4-(D,L-N-Benzoylarginylamido)-phenylimino)-1-methyl-2-phenyl-indolizinium-Sulfat

N-Benzoyl-D,L-arginin-4-nitranilid wird analog Beispiel 3 über Palladium/Kohle zur Aminoverbindung hydriert, die analog Beispiel 1a) zur Nitrosoverbindung oxidiert wird. Wie im Beispiel 4c) beschrieben, wird die Nitrosoverbindung mit 1-Methyl-2-phenylindolizin umgesetzt, wobei die Nitrosoverbindung in zweifachem überschuß eingesetzt und die Reaktionszeit auf 15 Minuten begrenzt wird. Man erhält das folgende chromogene Peptidhydrolase-Substrat.

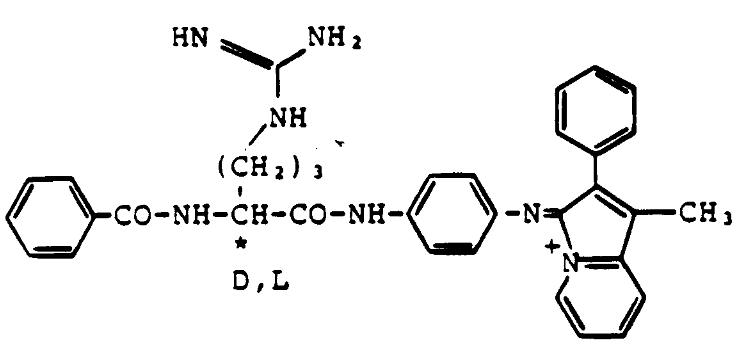

x $HSO_4^-$

$R_f$ = 0,38 (n-Butanol/Eisessig/Wasser 2:1:1).

**Beispiel 12:**

Herstellung von 1,2-Diphenyl-3-(4-L-alanylamido)-phenylimino-indolizinium-trifluoracetat

Analog Beispiel 7 erhält man aus BOC-L-alanin, N-Carbobenzoxyphenylen-diaminund 1,2-Diphenyl-indolizin 1,2-Diphenyl-3-(4-t-Butoxy-carbonyl-L-alanyl-amido)-phenylimido-indoliziniumacetat. Analog Beispiel 8 wird die BOC-Gruppe abgespalten und man erhält eine Verbindung der folgenden Struktur.

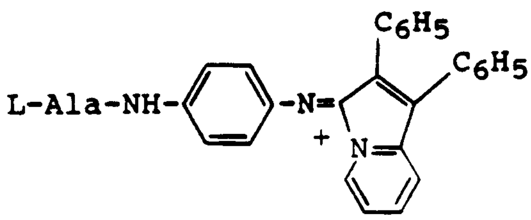

x $CF_3COO^-$ · $CF_3COOH$

Fp 114 - 116 °C (Zers.)
$R_f$ = 0.2 (n-Butanol/Eisessig/Wasser, 2:1:1)

**Beispiel 13:**

Herstellung von 2H-1,4-Dihydro-1,4-dimethyl-2-oxo-3-(4-(L-alanylamido)-2-(N-isopropylamidocarbonyl)-phenyl-imino)-pyrrolo-[3.2-b]-pyridinium-bis-trifluoracetat

a) 5.46 g 5-Amino-2-nitrobenzoesäure und 4.6 g 1-Hydroxybenzotriazol-Hydrat werden in 30 ml Dimethylformamid gelöst und die Lösung bei ca. 10 - 15 °C mit einer Lösung von 6.18 g Dicyclohexylcarbodiimid versetzt. Nach einer Stunde bei Raumtemperatur werden 2.6 ml Isopropylamin unter leichter Kühlung zugesetzt. Das Gemisch wird noch 2 Stunden bei Raumtemperatur gerührt und nach dem Stehenlassen über Nacht aufgearbeitet. Der Niederschlag wird abfiltriert und das Filtrat eingeengt. Der Rückstand wird in Essigsäureethylester aufgenommen und die Lösung rasch mit Natriumhydrogencarbonat-Lösung gewaschen. Der aus der Essigesterphase ausfallende Niederschlag wird isoliert und getrocknet. Man erhält 5.5 g 2-(N-Isopropylamidocarbonyl)-4-nitro-anilin vom Fp 211 °C.

b) 4.4 g BOC-L-alanin, 5.2 g der unter a) erhaltenen Verbindung und 1.77 Imidazol werden in 117 ml Pyridin gelöst und bei -20 °C mit 3.4 ml Phosphoroxychlorid versetzt. Nach der Zugabe läßt man langsam auf Raumtemperatur aufwärmen, destilliert das Pyridin ab, nimmt den Rückstand in Natriumhydrogencarbonat-Lösung auf und extrahiert mit Essigsäureethylester. Der Essigesterextrakt wird mit Natriumhydrogencarbonat-Lösung und wässriger Citronensäurelösung (5 Gew.-%) gewaschen, getrocknet und eingedampft. Das Rohprodukt wird durch Chromatographie an Kieselgel mit Ligroin-Aceton (2:1) als Laufmittel gereinigt. Die Produktfraktionen werden aus Diethylether/Ligroin umkristallisiert. Man erhält 3.2 g BOC-L-alanyl-2-(N-isopropylamidocarbonyl)-4-nitroanilid vom Fp 125 - 129 °C (Zers.).

c) Die unter b) erhaltene Verbindung wird in 150 ml Ethanol und 50 ml Wasser über einem Palladium auf Kohle Katalysator hydriert. Das Reaktionsprodukt wird analog Beispiel 4 b) zur Nitrosoverbindung oxidiert und analog Beispiel 4 c) mit 2H-1,4-Dihydro-1,4-dimethyl-pyrrolo-[3,2-b]-pyridin-2-on umgesetzt. Die Abspaltung der BOC-Schutzgruppe wird analog Beispiel 4 d) durchgeführt und man erhält die Titelverbindung der folgenden Struktur.

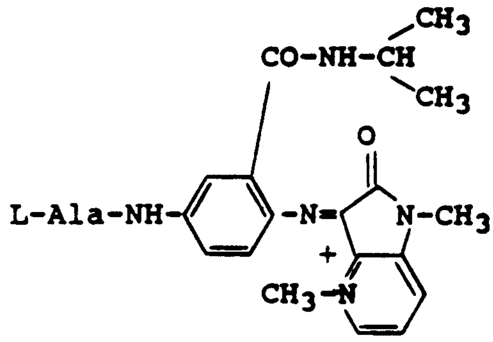

x $CF_3COOO-$
x $CF_3COOH$

$R_f$ = 0.1 (n-Butanol/Eisessig/Wasser 2:1:1)
Fp 124 °C (Zers.)

**Beispiel 14:**

Herstellung von 2H-1,4-Dihydro-1,4-dimethyl-2-oxo-3-(4-(N-tert.butoxy-D-phenylalanyl-L-prolyl-L-arginyl-amido)-phenylimino)-pyrrolo-[3,2-b]-pyridinium-sulfat

a) Synthese von N-Carbobenzoxy-phenylendiamin
Analog Beispiel 7 a) erhält man aus 60 g p-Nitranilin und 204 ml Chlorameisensäurebenzylester insgesamt 109.9 g N-Benzyloxycarbonyl-4-nitranilid ($R_f$ = 0.75 Kieselgel, Toluol-Dioxan-Eisessig 72:20:8), das in 1090 ml Tetrahydrofuran gelöst, mit 1900 ml 2 N Natronlauge und 436 g Natriumdithionit versetzt wird. Zur Vervollständigung der Reduktion gibt man nochmals eine Lösung von 100 g Natriumdithionit in 1000 ml Wasser zu. Die Reaktionsmischung wird mit Wasser verdünnt und das Produkt mit Essigsäureethylester extrahiert. Das Rohprodukt wird aus Essigester : Ligroin (2:1) umkristallisiert. Man erhält 57.3 g der Titelverbindung $R_f$ = 0.3 (Kieselgel, Toluol-Dioxan-Eisessig 72:20:8)
b) BOC-L-prolyl-(Nω-nitroarginyl)-4-benzyloxycarbonyl-amido-anilid
18 g (443 mmol) BOC-L-Pro-(Nω-nitro-Arg)-OH, 13.15 g (85.8 mmol) N-Hydroxybenzotriazol-Hydrat und 10.9 g (45.1 mmol) N-Carbobenzoxyphenylendiamin werden in insgesamt 240 ml Dimethylformamid gelöst. Die Lösung wird auf -10 °C gekühlt und tropfenweise mit einer Lösung von 9.7 g (47 mmol) Dicyclohexylcarbodiimid in 70 ml Dimethylformamid versetzt. Danach rührt man das Gemisch 1 Stunde bei 0 °C und anschließend bei Raumtemperatur. Nach dem Stehenlassen über Nacht wird vom Dicyclohexylharnstoff abgesaugt, die Dimethylformamid-Lösung auf etwa die Hälfte eingeengt, mit etwa dem gleichen Volumen Wasser versetzt und das Gemisch zweimal mit Essigsäureethylester extrahiert. Die vereinigten Extrakte werden mit wässriger Citronensäurelösung (5 Gew.-%) oder 5 % Kaliumhydrogensulfat-Lösung, Wasser, 5 %iger Natriumhydrogencarbonat-Lösung und Wasser gewaschen. Die Essigesterphase wird eingedampft, der Rückstand in Aceton gelöst und nach einigem Stehen von restlichem Dicyclohexylharnstoff abgesaugt. Das Filtrat wird eingedampft und der Rückstand aus Essigsäureethylester umkristallisiert. Die Substanz kristallisiert sehr langsam. Nach 2 - 3tägigem Stehen werden die Kristalle abgesaugt. Ausbeute 19.09 g (69 %).
Zur Reinigung wird die Substanz noch über Kieselgel mit $CH_3OH$ chromatographiert.
$R_f$ = 0.8 (Kieselgel, Aceton/Methylenchlorid/Eisessig 50:45:5)
c) 24.3 g des unter b) erhaltenen geschützten Dipeptids werden in 200 ml Tetrahydrofuran gelöst und unter Rühren mit 200 ml etherischer Salzsäure (ca. 5 n) versetzt.
Es fällt ein klebriger Niederschlag aus, der beim Rühren bei Raumtemperatur (1.5 Stunden) kristallisiert. Der Niederschlag wird abgesaugt, gut mit Diethylether gewaschen und im Vakuum-Trockenschrank bei 50 °C getrocknet.
Man erhält 22.4 g (100 %) der Titelverbindung.
$R_f$ = 0.55 (Kieselgel, Essigsäureethylester/Aceton/Eisessig/Wasser 50:25:12.5:12.5).
d) BOC-D-Phenylalanyl-L-prolyl-Nω-nitro-L-arginyl-(4-benzyloxy-carbonylanilid)
4.64 g BOC-D-Phenylalanin und 5.05 g der unter c) erhaltenen Verbindung werden in 52 ml Dimethylformamid gelöst und auf 0 °C gekühlt. Man versetzt die Lösung mit 5.25 ml N-Methylmorpholin und 7.7 g einer 50 Gewichtsproz. Lösung von n-Propanphosphonsäureanhydrid in Methylenchlorid. Die Mischung wird über Nacht bei Raumtemperatur stehengelassen. Die Reaktionsmischung wird eingedampft. Den Rückstand nimmt man in Essigsäureethylester auf und wäscht die organische Phase mit 5proz.

Natriumhydrogencarbonat-Lösung, Wasser und 0.5 n Salzsäure. Die organische Phase wird getrocknet und eingedampft. Man erhält 4.92 g öliges Produkt.
$R_f$ = 0.45 (Kieselgel, Methylenchlorid-Methanol (95:5)
e) BOC-D-phenylalanin-L-prolyl-L-arginyl-(4-aminoanilid)
9.6 g der unter d) erhaltenen Verbindung werden in 250 ml Ethanol, 25 ml Wasser und 15 ml Eisessig gelöst und über 2.4 g eines Palladium auf Kohle Katalysators bei ca. 6 bar hydriert. Der nach dem Abfiltrieren des Katalysators und Eindampfen des Filtrats verbleibende Rückstand wird mit Diethylether kristallin. Man erhält 4.93 g BOC-D-phenylalanyl-L-prolyl-L-arginyl-(4-amino-anilid) vom Fp 133 - 135 °C (Z), $R_f$ = 0.6 Essigsäureethylester/Aceton/Eisessig/Wasser (50:25:12.5:12.5).
f) Analog Beispiel 1 a) wird die oben unter e) erhaltene Verbindung zur Nitrosoverbindung oxidiert und analog Beispiel 1 c) mit 2H-1,4-Dihydro-1,4-dimethyl-pyrrolo-[3,2-b]-pyridin-2-on umgesetzt. Man erhält eine Verbindung der folgenden Struktur,

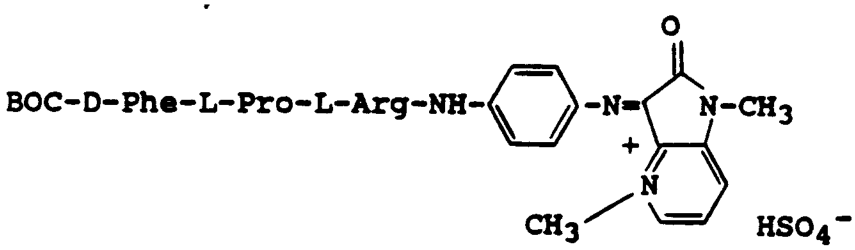

die durch Chromatographie an Kieselgel mit n-Butanol/Eisessig/Wasser (2:1:1) gereinigt wird. Fp 175 °C (Zers.), $R_f$ = 0.38 (Kieselgel, n-Butanol/Eisessig/Wasser, 2:1:1)

**Beispiel 15:**

Herstellung von 2H-1,4-Dihydro-1,4-dimethyl-2-oxo-3-(4-(D-phenylalanyl-L-prolyl-L-arginylamido)-phenyl-imino)-pyrrolo-(3,2-b)-pyridinium-sulfat-trifluoracetat

Die Verbindung aus Beispiel 14 wird analog Beispiel 4 d) zur Abspaltung der BOC-Gruppe mit Trifluoressigsäure behandelt. Man erhält eine Verbindung der folgenden Struktur.

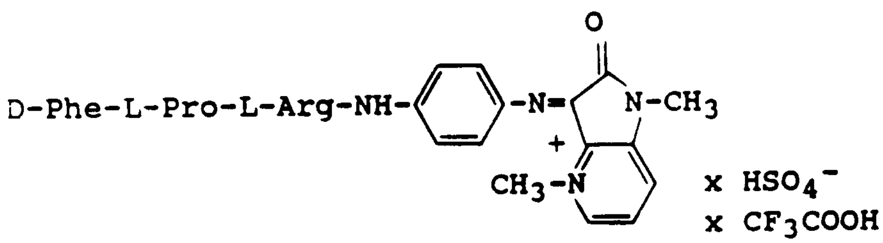

Fp 185 °C (Zers.), $R_f$ = 0.15 (n-Butanol-Eisessig-Wasser (2:1:1)).

**Beispiel 16:**

Enzymatische Spaltung der erfindungsgemäßen Verbindungen

Beispielhaft wird anhand einiger Verbindungen der Nachweis Peptidbindungen spaltender Enzyme gezeigt.

Thrombin

Die Substanz aus Beispiel 1c) und die Substanzen aus Beispiel 2b), 2c), 2d), 2e), 2f), 2g), 2h), 2i), 2k), 14) und 15) wurden in jeweils 0,1 mol/l Hepes-Puffer, pH 7,5, gelöst und mit Thrombin versetzt.
Die Absorptionsmaxima für die erfindungsgemäßen Verbindungen vor und nach der Spaltung sind der folgenden Zusammenstellung zu entnehmen:

| Substanz aus Beispiel | λmax in nm vor der Spaltung | λmax in nm nach der Spaltung |
|---|---|---|
| 1c) | 526 | 544 |
| 2a) | 515 | 566 |
| 2b) | 501 | 562 |
| 2c) | 525 | 558 |
| 2d) | 342 mit Schulter bei 420 | 568 |
| 2e) | 470 | 584 |
| 2f) | 464 | 588 |
| 2g) | 528 | 564 |
| 2h) | 529 | 564 |
| 2i) | 449 | 540 |
| 2k) | 530 | 567 |
| 14) | 525 | 558 |
| 15) | 525 | 558 |

Gerinnungsfactor Xa

Die Substanz aus Beispiel 3 wurde in 0,1 mol/l Hepes-Puffer, pH 7,5 gelöst und mit dem Gerinnungsfactor Xa aus Rind umgesetzt. Die Messungen ergaben ein Absorptionsmaximum von 532 nm vor und 564 nm nach der Spaltung.

γ-Glutamyltranspeptidase

Die Substanz aus Beispiel 4c) wurde in Tris-Puffer 0,1 mol/l pH 7,5 gemeinsam mit 50 mol/l Glyclglycin gelöst und mit Glutamyltranspeptidase umgesetzt. Die Messungen ergaben ein Absorptionsmaximum von 525 nm vor und 558 nm nach der Spaltung.

Chymotrypsin

Die Substanzen aus Beispiel 5 und 6 wurden jeweils in 0,2 mol/l Tris-Puffer, pH 8,1, 25 mol/l NaCl unter Zugabe von Calciumionen gelöst und mit Chymotrypsin umgesetzt. Es wurden die Absorptionsmaxima vor und nach der Spaltung ermittelt.

| | |
|---|---|
| Substanz aus Beispiel 5: | Vor der Spaltung 525 nm,<br>nach der Spaltung 558 nm. |
| Substanz aus Beispiel 6: | Vor der Spaltung 366 nm mit einer Schulter bei 485 nm,<br>nach der Spaltung 594 nm. |

Aminopeptidase M:

Die Substanzen aus Beispiel 8, 10, 12 und 13 wurden jeweils in 0,2 mol/l Tris-Puffer, pH 8,1, gelöst und mit Aminopeptidase M umgesetzt. Es wurden die Absorptionsmaxima vor und nach der Spaltung ermittelt.

| Substanz aus Beispiel | $\lambda_{max}$ in nm vor der Spaltung | $\lambda_{max}$ in nm nach der Spaltung |
|---|---|---|
| 8 | 512 | 558 |
| 10 | 544 | 566 |
| 12 | 446 | 605 |
| 13 | 530 | 567 |

Subtilisin

Die Substanz aus Beispiel 9b) wurde in 0,2 mol/l Tris-Puffer, pH 8,1 gelöst und mit Subtilisin umgesetzt. Die Messungen ergaben ein Absorptionsmaximum von 548 nm vor und 566 nm nach der Spaltung.

Trypsin

Die Substanz aus Beispiel 11 wurde in 200 mol/l Tris/HCl, pH 8,1, gelöst und mit Trypsin umgesetzt. Die Messungen ergaben vor der Spaltung ein Absorptionsmaximum bei 418 nm mit einer Schulter bei 525 nm und nach der Spaltung ein Absorptionsmaximum von 594 nm.

**Beispiel 17:**

Bestimmung der Einphasengerinnungszeit nach Quick

Herstellung des Reagenzes

58 mg der Substanz aus Beispiel 2 c) werden in 100 ml 0.1 mol/l Hepes-Puffer pH 7,0 gelöst und mit 36 ml Thromboplastin, hergestellt nach EP-B-0 038 773, gemischt. Diese Lösung wird in Portionen zu 3 ml in 35 ml Fläschchen gefüllt und lyophilisiert.

Das Lyophilisat eines Fläschchens wird in 30 ml 0.1 mol/l Hepes-Puffer pH 7,0 der 6 mmol/l Calciumchlorid enthält, gelöst.

Messung der Quick-Zeit

In einem Photometer mit temperierbarem Küvettenhalter und angeschlossenem Schreiber werden die Messungen bei 37 °C und bei einer Wellenlänge von 560 nm durchgeführt. 1 ml Reagenz wird auf 37 °C vortemperiert und in einer 1 cm Kunststoffküvette vorgelegt. Dazu pipettiert man 0.1 ml Citratplasma, mischt sofort und startet gleichzeitig den Schreiber. Es wird die Zeit gemessen, bis eine Extinktionszunahme von 0.1 E erfolgt.

Nach diesem Meßverfahren wurde eine Bezugskurve mit Poolplasmaverdünnungen ermittelt. Die Ergebnisse werden in der folgenden Tabelle wiedergegeben:

| Quick % der Proben | Zeit in Sekunden bis zu einer Extinktionsänderung von 0.1 E |
|---|---|
| 100 % | 45.9 |
| 50 % | 62.4 |
| 33 % | 76,7 |
| 25 % | 91,6 |
| 20 % | 108,1 |
| 12,5 % | 156,4 |
| 10 % | 197,9 |

Ein Vergleich, der nach dem erfindungsgemäßen Verfahren und nach dem von U. Becker et al. in Haemostasis 12, 1 - 2, 73 (1982) beschriebenen Verfahren, bei dem statt der Substanz aus Beispiel 2 c), Tos-Gly-Pro-Arg-p-Nitroanilinacetat als Indikator verwendet wird, ergab für Plasmen von antikoagulierten Patienten eine Übereinstimmung mit einem Korrelationsfactor von 0.988.

**Beispiel 18:**

Bestimmung der Einphasengerinnungszeit nach Quick mittels remissionsphotometrischer Messung

Indikatornetz

Ein Nylongewebe Typ NY75HC der Firma Züricher Beuteltuchfabrik, Zürich, Schweiz, wird mit einer Lösung bestehend aus
2,5 g Mowiol 26/88 der Firma Hoechst AG Deutschland,
150 mg Substanz aus Beispiel 2c) und
110 mg wasserfreiem Calciumchlorid ad 100 ml Wasser,
imprägniert und anschließend bei 50° C im Trockenschrank getrocknet.

Thromboplastinnetz

Ein Nylonnetz Typ NY75HC der Firma Züricher Beuteltuchfabrik, Zürich, Schweiz, wurde mit einer Thromboplastinlösung der unten beschriebenen Zusammensetzung imprägniert und anschließend bei 50° C im Trockenschrank getrocknet.

Die Thromboplastinlösung wurde folgendermaßen hergestellt:
3,5 g Mowiol 18/88 der Firma Hoechst AG, Deutschland werden zusammen mit 22 ml einer Thromboplastinlösung, mit der man bei einem 100 % Plasma eine Quick-Zeit von 11, 0 - 12,6 sec. erzielt, in 100 ml 100 mmol/l Hepes-Puffer, pH 7,45 gelöst.

Indikatornetz und Thromboplastinnetz werden zu Streifen von 12 mm Breite geschnitten und zusammen mit 16 mm breiten Glasfaservliesen gemäß EP-A 0 208 218 und DE-A 36 10 429 zu einem Testträger nach DE-A 36 16 496 verarbeitet, wie er in Figur 1 dargestellt ist.

Der in Figur 1 dargestellte Testträger hat die prinzipielle Form eines konventionellen Teststreifens. Auf einer 100 mm breiten Polystyrolfolie (1) wird ein Glasfaservlies (4) gemäß EP-A 0 208 218 oder DE-A 36 10 429 in einer Breite von 16 mm gelegt und mit einem groben Nylonnetz (3) teilweise abgedeckt und an einem Ende mit einem Kleber (2) fixiert. Am freien Ende des Glasfaservlieses werden das Thromboplastinnetz (5) und das Indikatornetz (6) übereinander gelegt und mit einer 200 µm dicken transparenten Polycarbonatfolie (7) von 16 mm Breite abgedeckt und mit einem Kleber (8) festgeklebt. Die so hergestellten Bänder werden zu 6 mm breiten Streifen geschnitten.

Zur Durchführung des Quick-Tests wird Blut oder Plasma auf das Abdecknetz 3 aufgegeben und über die Glasfaserschicht 4 in den Bereich des Thromboplastinnetzes 5 und des Indikatornetzes 6 transportiert. Im Falle der Auftragung von Vollblut findet hierbei eine Abtrennung des Plasmas statt, wie dies in der europäischen Patentanmeldung EP-A 0 045 476 beschrieben ist. Um die Reaktion mit den auf den

Schichten 5 und 6 enthaltenen Reagenzien einzuleiten, werden diese nach unten gedrückt, so daß die Probenflüssigkeit in diese Schichten eindringt und die entsprechenden Reaktionen ablaufen.

Nähere Einzelheiten über die bei einem Gerinnungstest der vorliegenden Art ablaufenden chemischen Reaktionen sind der europäischen Patentanmeldung mit der Veröffentlichungsnummer EP-A 0 182 373 zu entnehmen.

Mit Reagenzträgern nach Figur 1 wurde auf dem Remissionsphotometer Reflotron® der Firma Boehringer Mannheim GmbH, Deutschland mit der LED für 567 nm mit Plasmaverdünnungen eine Eichkurve ermittelt. Hierzu wurden die Plasmen mit physiologischer Kochsalzlösung, der 10% Rinderserumalbumin zugegeben worden waren, auf unterschiedlich Quick-Prozente verdünnt. Die so vorbehandelten Proben wurden mit phyisiologischer Kochsalzlösung im Verhältnis 1 Teil Probe zu 6 Teile Kochsalzlösung verdünnt und 32 µl hiervon auf die Auftragsseite (Abdecknetz 3 in Figur 1) pipettiert.

Die Ergebnisse der Ermittlung einer Bezugskurve sind der folgenden Tabelle zu entnehmen:

| Quick % der Probe vor der endgültigen Verdünnung | Zeit in Sekunden bis zu einer Remissionsänderung von 10 % Remission |
|---|---|
| 100 % | 38,0 sec. |
| 50 % | 57,0 sec. |
| 33 % | 74,5 sec. |
| 25 % | 93,9 sec. |
| 20 % | 110,0 sec. |
| 12,5 % | 163,8 sec. |

**Patentansprüche**

1. Verbindungen der allgemeinen Formel II

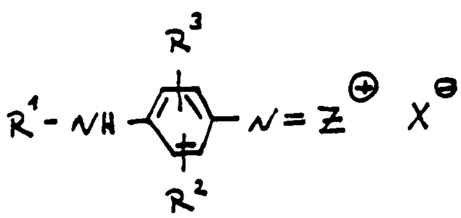

(II)

in der

R¹ einen Aminosäurerest oder den Rest eines Oligopeptids darstellt, in dem Aminogruppen gegebenenfalls durch Schutzgruppen substituiert sind,

$R^2$, $R^3$ die gleich oder verschieden sind, Wasserstoff, eine Niederalkyl-, Niederalkoxy-, Carboxy-, Niederalkoxycarbonyl-oder eine gegebenenfalls durch Niederalkyl substituierte Carboxamidogruppe bedeuten oder falls $R^2$ und $R^3$ benachbart sind, eine -CH = CH-CH = CH-Gruppe darstellen,

Z ein durchkonjugiertes, ein positiv geladenes Stickstoffatom enthaltendes bicyclisches, heterocyclisches System der Formel III

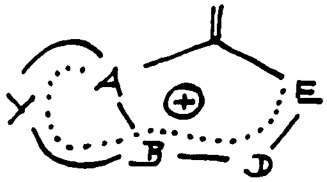

(III)

darstellt, in der

| | |
|---|---|
| A, B, D und E | ein Kohlenstoff- oder Stickstoffatom bedeuten, wobei sofern sie keine Brückenkopfatome sind, Kohlenstoffatome gegebenenfalls durch Sauerstoff, eine Niederalkyl-, Niederalkoxy, Aryl-, Aralkyl- oder Aryloxygruppe und Stickstoffatome gegebenenfalls durch eine Niederalkyl-, Aralkyl- oder Arylgruppe substituiert sind und |
| Y | eine ungesättigte Kohlenwasserstoffkette mit 3 - 5 Gliedern bedeutet, die gegebenenfalls durch Stickstoff-oder Schwefelatome unterbrochen ist oder solche Heteroatome am Anfang oder Ende der Kette enthält, wobei Kohlenstoffatome gegebenenfalls durch eine Niederalkyl-, Niederalkoxy-, Aralkyl-, Aryloxy- oder Arylgruppe und/oder Stickstoffatome gegebenenfalls durch eine Niederalkyl-, Aralkyl- oder Arylgruppe substituiert sind, zwei benachbarte Niederalkylreste gegebenenfalls eine gesättigte Alkylengruppe mit 3 - 5 Kohlenstoffatomen bilden und insgesamt die Summe der Heteroatome im Bicyclus maximal 3 ist und |
| X | ein Anion einer organischen oder anorganischen Säure bedeutet. |

**2.** Verbindungen gemäß Anspruch 1, dadurch gekennzeichnet, daß Z

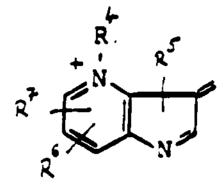

(IV)

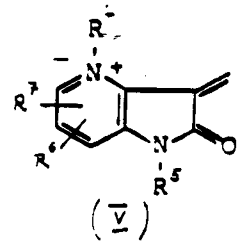

(V)

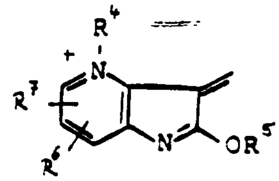

(VI)

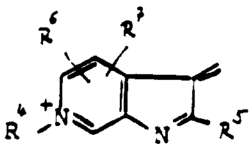

(VII)

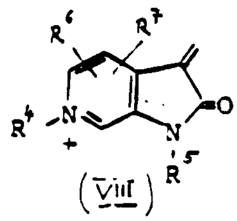

(VIII)

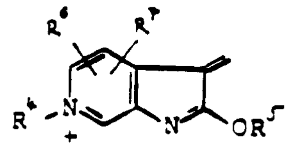

(IX)

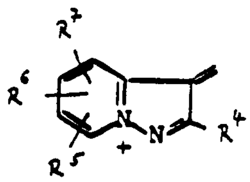

(X)

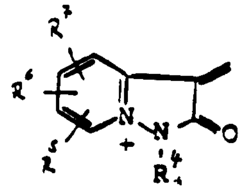

(XI)

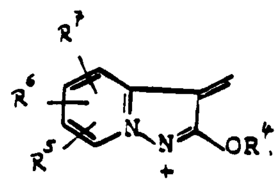

(XII)

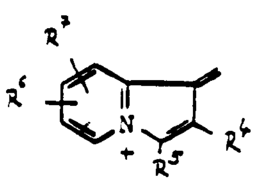

(XIII)

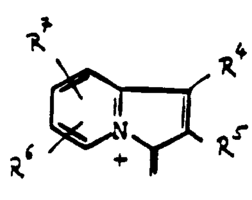

(XIV)

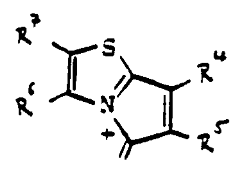

(XV)

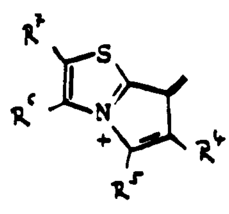

XVI

bedeutet und

$R^4$ - $R^7$, die gleich oder verschieden sein können, Wasserstoff, eine Niederalkyl-, Niederalkoxy-, Aralkyl-, Aryloxy- oder Arylgruppe darstellen.

**3.** Verfahren zur Herstellung von Verbindungen gemäß Anspruch 1, dadurch gekennzeichnet, daß man Verbindungen der allgemeinen Formel Ia)

$R^1$-OH (Ia)

in der

$R^1$ einen Aminosäurerest oder den Rest eines Oligopeptids darstellt, in dem Aminogruppen durch Schutzgruppen substituiert sind,

mit

a) Phenylendiaminderivaten der allgemeinen Formel XXXIII

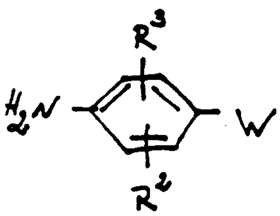

(XXXIII)

in der

$R^2$ und $R^3$, die gleich oder verschieden sind, Wasserstoff, eine Niederalkyl-, Niederalkoxy-, Carboxy-, Niederalkoxycarbonyl-oder eine gegebenenfalls durch Niederalkyl substituierte Carboxamidogruppe oder falls $R^2$ und $R^3$ benachbart sind, eine -CH = CH-CH = CH-Gruppe und

W eine durch eine Schutzgruppe substituierte Aminogruppe darstellt,

umsetzt, anschließend die Aminoschutzgruppe der Gruppe W abspaltet und die hieraus resultierende Verbindung mit freier $NH_2$-Gruppe oxidiert oder

b) p-Nitroanilinderivaten der allgemeinen Formel XXXIV

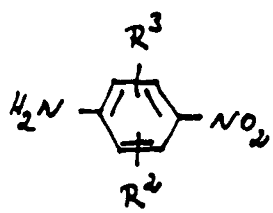

(XXXIV)

in der

$R^2$, $R^3$ die oben angegebene Bedeutung haben,

umsetzt, die hieraus resultierende Nitroverbindung zum entsprechenden Hydroxylamin reduziert und dann oxidiert und die so erhaltenen Nitrosoverbindungen der allgemeinen Formel XVII

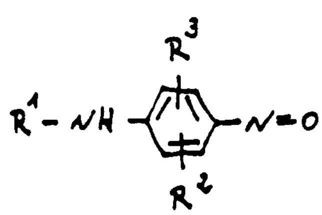

(XVII)

in der

$R^1$-$R^3$ die oben angegebene Bedeutung haben unter saurer Katalyse mit Verbindungen der allgemeinen Formel XVIII

Z'-H (XVIII)

in der

Z' ein ungeladenes, mindestens ein Stickstoffatom enthaltendes bicyclisches, heterocyclisches System der allgemeinen Formel (XIXa) oder (XIXb) darstellt,

(XIXa) 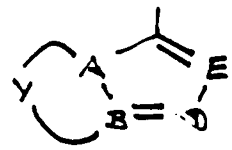 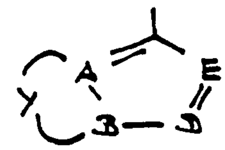 (XIXb)

in denen

A, B, D und E jeweils ein Kohlenstoff- oder Stickstoffatom bedeuten, wobei sofern sie keine Brückenkopfatome sind Kohlenstoffatome gegebenenfalls durch Sauerstoff, eine Niederalkyl-, Niederalkoxy-, Aryl-, Aralkyl- oder Aryloxygruppe und Stickstoffatome gegebenenfalls durch eine Niederalkyl-, Aralkyl- oder Arylgruppe substituiert sind,

Y eine ungesättigte Kohlenwasserstoffkette mit 3 - 5 Gliedern bedeutet, die gegebenenfalls durch Stickstoff-oder Schwefelatome unterbrochen ist oder solche Heteroatome am Anfang oder Ende der Kette enthält,

wobei Kohlenstoffatome gegebenenfalls durch eine Niederalkyl-, Niederalkoxy-, Aralkyl-, Aryloxy- oder Arylgruppe und/oder Stickstoffatome gegebenenfalls durch eine Niederalkyl-, Aralkyl- oder Arylgruppe substituiert sind, zwei benachbarte Niederalkylreste gegebenenfalls eine gesättigte Alkylengruppe mit 3 - 5 Kohlenstoffatomen bilden,

insgesamt die Summe der Heteroatome im Bicyclus maximal 3 ist, Doppelbindungen der ungesättigten Kette Y in Konjugation zum Doppelbindungssystem des Fünfringes stehen und/oder wenn A oder B ein Stickstoffatom ist, dieses Enaminstrukturen zu beiden Ringsystemen ausbildet, eingesetzt werden,

umsetzt und anschließend Aminoschutzgruppen gegebenenfalls vollständig oder teilweise entfernt.

**4.** Verbindungen der allgemeinen Formel XVII

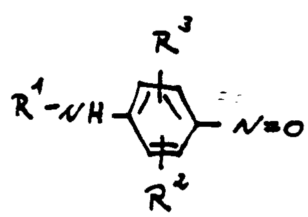 (XVII)

in der

$R^1$ einen Aminosäurerest oder den Rest eines Oligopeptids, in dem Aminogruppen gegebenenfalls durch Schutzgruppen substituiert sind und

$R^2$, $R^3$, die gleich oder verschieden sind, Wasserstoff, eine Niederalkyl-, Niederalkoxy-, Carboxy-, Niederalkoxycarbonyl-oder eine gegebenenfalls durch Niederalkyl substituierte Carboxamidogruppe, oder falls $R^2$ oder $R^3$ benachbart sind, eine -CH = CH-CH = CH-Gruppe

bedeuten.

**5.** Verfahren zur Herstellung von Verbindungen der allgemeinen Formel XVII

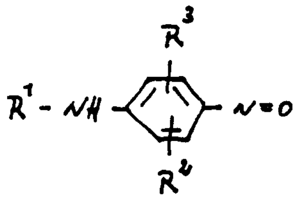

in der

$R^1$ einen Aminosäurerest oder den Rest eines Oligopeptids, in dem Aminogruppen gegebenenfalls durch Schutzgruppen substituiert sind und

$R^2$ und $R^3$, die gleich oder verschieden sind, Wasserstoff, eine Niederalkyl-, Niederalkoxy-, Carboxy-, Niederalkoxycarbonyl- oder eine gegebenenfalls durch Niederalkyl substituierte Carboxamidogruppe, oder falls $R^2$ und $R^3$ benachbart sind, eine -CH = CH-CH = CH-Gruppe,

bedeuten, dadurch gekennzeichnet, daß eine Verbindung de allgemeinen Formel I a)

$R^1$-OH (Ia)

in der

$R^1$ einen Aminosäurerest oder den Rest eines Oligopeptids darstellt, in dem Aminogruppen durch Schutzgruppen substituiert sind,

mit

a) einem Phenyldiaminderivat der allgemeinen Formel XXXIII

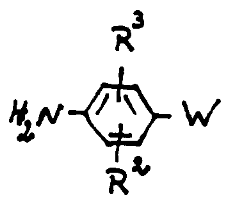

in dem

$R^2$ und $R^3$ die oben angegebene Bedeutung haben und

W eine durch eine Schutzgruppe substituierte Aminogruppe darstellt, umgesetzt wird, anschließend die Aminoschutzgruppe der Gruppe W abgespalten und die hieraus resultierende Verbindung mit freier $NH_2$-Gruppe oxidiert wird oder

b) einem p-Nitroanilinderivat der allgemeinen Formel XXXIV

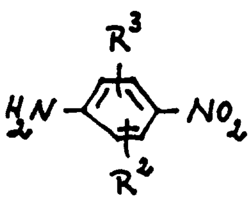

in dem

$R^2$ und $R^3$ die oben angegebene Bedeutung haben,

umgesetzt wird, die hieraus resultierende Nitroverbindung zum entsprechenden Hydroxylamin oder Amin reduziert und dann oxidiert wird.

6. Verwendung von Verbindungen gemäß Anspruch 4 zur Herstellung von Verbindungen gemäß den Ansprüchen 1 - 2.

7. Verwendung von Verbindungen gemäß den Ansprüchen 1 - 2 zur Bestimmung von Peptidbindungen spaltenden Enzymen.

8. Verwendung von Verbindungen gemäß Anspruch 7 zur Bestimmung von Gerinnungsparametern.

9. Diagnostisches Mittel zur Bestimmung von Peptidbindungen spaltenden Enzymen enthaltend ein oder mehrere chromogene Substrate, eine geeignete Puffersubstanz sowie gegebenenfalls weitere üblicherweise verwendete Hilfsstoffe, dadurch gekennzeichnet, daß es als chromogene Substrate Verbindungen der Ansprüche 1 - 2 enthält.

**Claims**

1. Compounds of the general formula II

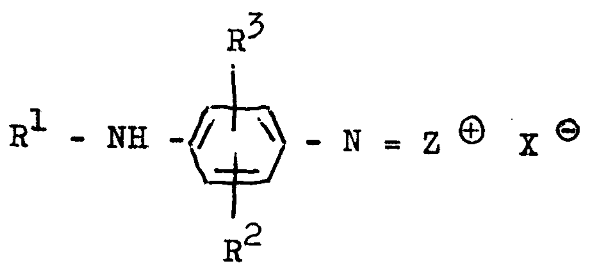

(II)

in which $R^1$ represents an amino acid residue or the residue of an oligopeptide in which amino groups are possibly substituted by Protective groups, $R^2$ and $R^3$, which are the same or different, signify hydrogen, a lower alkyl, lower alkoxy, carboxyl, lower alkoxycarbonyl group or a carboxamido group possibly substituted by lower alkyl or, if $R^2$ and $R^3$ are adjacent, represent a -CH = CH-CH = CH- group, Z represents a conjugated-through bicyclic heterocyclic system, containing a positively-charged nitrogen atom, of the formula III

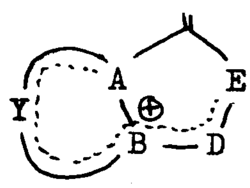

(III)

in which A, B, D and E signify a carbon or nitrogen atom, whereby, insofar as they are not bridgehead atoms, carbon atoms are possibly substituted by oxygen, a lower alkyl, lower alkoxy, aryl, aralkyl or aryloxy group and nitrogen atoms possibly substituted by a lower alkyl, aralkyl or aryl group and Y signifies an unsaturated hydrocarbon chain with 3 - 5 members which is possibly interrupted by nitrogen or sulphur atoms or contains such heteroatoms at the beginning or end of the chain, whereby carbon atoms are possibly substituted by a lower alkyl, lower alkoxy, aralkyl, aryloxy or aryl group and/or nitrogen atoms possibly by a lower alkyl, aralkyl or aryl group, two adjacent lower alkyl radicals possibly form a saturated alkylene radical with 3 - 5 carbon atoms and, in all, the sum of the heteroatoms in the bicyclic radical is maximum 3 and X signifies an anion of an organic or inorganic acid.

**2.** Compounds according to claim 1, characterised in that Z signifies

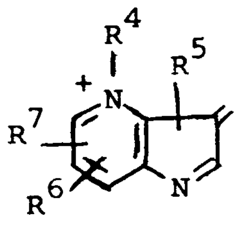

(IV)

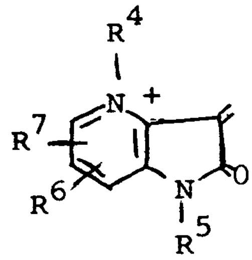

(V)

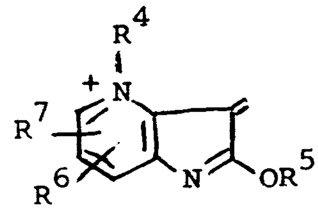

(VI)

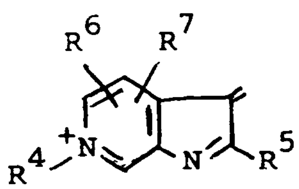

(VII)

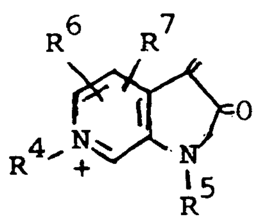

(VIII)

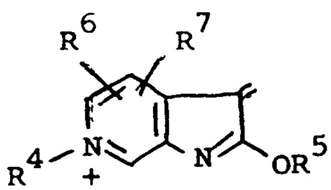

(IX)

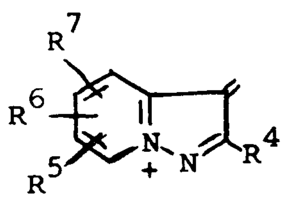

(X)

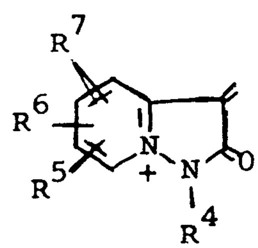

(XI)

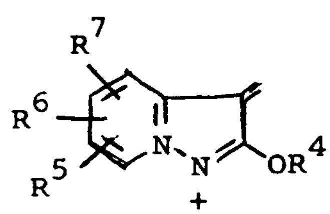

(XII)

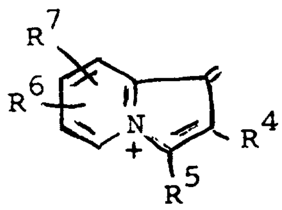

(XIII)

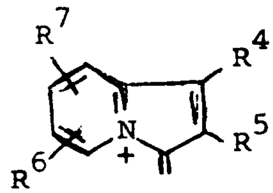

(XIV)

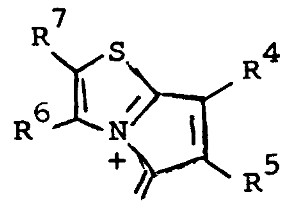

(XV)

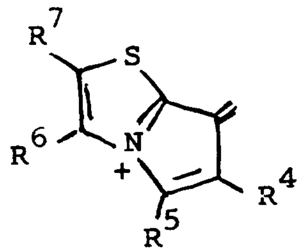

(XVI)

and $R^4$ - $R^7$, which can be the same or different, represent hydrogen, a lower alkyl, lower alkoxy, aralkyl, aryloxy or aryl group.

**3.** Process for the preparation of compounds according to claim 1, characterised in that one reacts compounds of the general formula Ia)

$R^1$ - OH (Ia)

in which $R^1$ represents an amino acid residue or the residue of an oligopeptide in which amino groups are substituted by protective groups, with

a) phenylenediamine derivatives of the general formula XXXIII

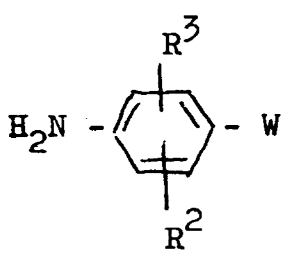

(XXXIII)

in which $R^2$ and $R^3$, which are the same or different, represent hydrogen, a lower alkyl, lower alkoxy, carboxyl, lower alkoxycarbonyl group or a carboxamido group possibly substituted by lower alkyl or, if $R^2$ and $R^3$ are adjacent, a -CH=CH-CH=CH- group and W an amino group substituted by a protective group, subsequently splits off the amino protective group of the group W and oxidises the compound with free $NH_2$ group resulting herefrom or

b) p-nitroaniline derivatives of the general formula XXXIV

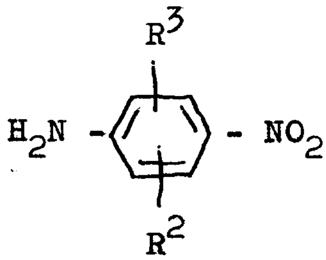

(XXXIV)

in which $R^2$ and $R^3$ have the above-given meaning, reduces the nitro compound resulting therefrom to the corresponding hydroxylamine and then oxidises and reacts the so-obtained nitroso compounds of the general formula XVII

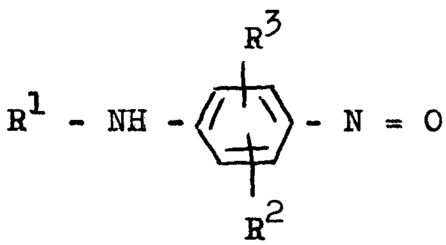

(XVII)

in which $R^1$ - $R^3$ have the above-given meanings, under acidic catalysis with compounds of the general formula XVIII

Z' - H (XVIII)

in which Z' represents an uncharged, bicyclic, heterocyclic system, containing at least one nitrogen atom, of the general formulae (XIXa) or (XIXb)

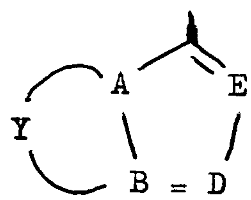

(XIXa)

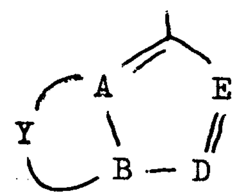

(XIXb)

in which A, B, D and E each signify a carbon or nitrogen atom, whereby, insofar as they are not bridgehead atoms, carbon atoms are possibly substituted by oxygen, a lower alkyl, lower alkoxy, aryl, aralkyl or aryloxy group and nitrogen atoms possibly substituted by a lower alkyl, aralkyl or aryl group, Y signifies an unsaturated hydrocarbon chain with 3 - 5 members which is possibly interrupted by nitrogen or sulphur atoms or contains such heteroatoms at the beginning or end of the chain, whereby carbon atoms are possibly substituted by a lower alkyl, lower alkoxy, aralkyl, aryloxy or aryl group and/or nitrogen atoms possibly substituted by a lower alkyl, aralkyl or aryl group, two adjacent lower alkyl radicals possibly form a saturated alkylene group with 3 - 5 carbon atoms, in all the sum of the heteroatoms in the bicyclic radical is maximum 3, double bonds of the unsaturated chain Y stand in conjugation with the double bond system of the five-membered ring and/or when A or B is a nitrogen atom, this forms enamine structures to both ring systems, and subsequently amino protective groups are possibly removed completely or partly.

**4.** Compounds of the general formula XVII

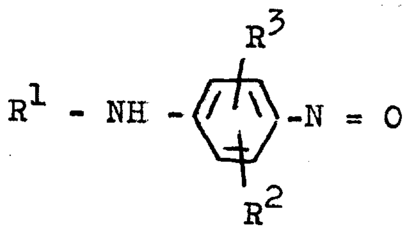 (XVII)

in which $R^1$ signifies an amino acid residue or the residue of an oligopeptide in which amino groups are possibly substituted by protective groups and $R^2$ and $R^3$, which are the same or different, signify hydrogen, a lower alkyl, lower alkoxy, carboxyl, lower alkoxycarbonyl group or a carboxamido group possibly substituted by lower alkyl or, if $R^2$ and $R^3$ are adjacent a -CH = CH-CH = CH- group.

**5.** Process for the preparation of compounds of the general formula XVII

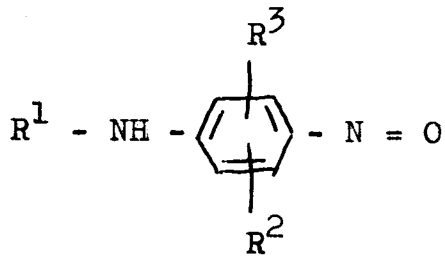

in which $R^1$ signifies an amino acid residue or the residue of an oligopeptide in which amino groups are possibly substituted by protective groups and $R^2$ and $R^3$, which are the same or different, signify hydrogen a lower alkyl, lower alkoxy, carboxyl, lower alkoxycarbonyl group or a carboxamide group possibly substituted by lower alkyl or, if $R^2$ and $R^3$ are adjacent, a -CH = CH-CH = CH- group, characterised in that a compound of the general formula Ia)

$R^1$ - OH (Ia)

in which $R^1$ represents an amino acid residue or the residue of an oligopeptide in which amino groups are substituted by protective groups is reacted with

a) a phenyldiamine derivative of the general formula XXXIII

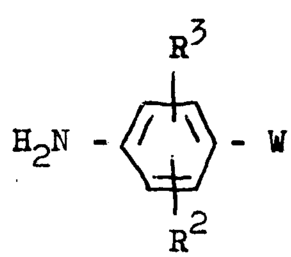

in which $R^2$ and $R^3$ have the above-given meaning and W represents an amino group substituted by a protective group, subsequently the amino protective group of the group W is split off and the compound with free $NH_2$ group resulting herefrom is oxidised or

b) is reacted with a p-nitroaniline derivative of the general formula XXXIV

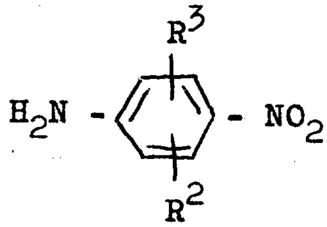

in which $R^2$ and $R^3$ have the above-given meaning, the nitro compound resulting herefrom is reduced to the corresponding hydroxylamine or amine and then oxidised.

**6.** Use of compounds according to claim 4 for the preparation of compounds according to claims 1 - 2.

**7.** Use of compounds according to claims 1 - 2 for the determination of peptide bond-cleaving enzymes.

**8.** Use of compounds according to claim 7 for the determination of coagulation parameters.

**9.** Diagnostic agent for the determination of peptide bond-cleaving enzymes containing one or more chromogenic substrates, a suitable buffer substance, as well as possibly further usually employed adjuvant materials, characterised in that, as chromogenic substrates, it contains compounds of claims 1 - 2.

**Revendications**

**1.** Composés de formule générale II

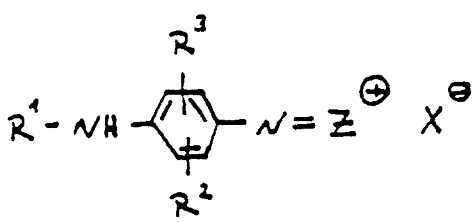

(II)

dans laquelle
$R^1$ représente un résidu d'aminoacide ou le résidu d'un oligopeptide, dans lequel les groupes amino sont éventuellement substitués par des groupes protecteurs,
$R^2$, $R^3$, qui peuvent être identiques ou différents, représentent un atome d'hydrogène, un groupe alkyle inférieur, alcoxy inférieur, carboxy, alcoxy(inférieur)carbonyle ou un groupe carboxamido éventuellement substitué par un groupe alkyle inférieur, ou dans le cas où $R^2$ et $R^3$ sont voisins, un groupe -CH = CH-CH = CH-,
Z représente un système conjugué hétérocyclique, bicyclique, contenant un atome d'azote chargé positivement, répondant à la formule III

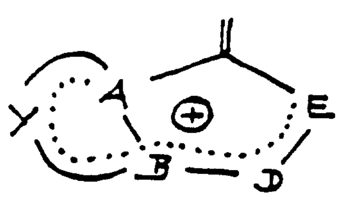

(III)

dans laquelle

A, B, D et E représentent un atome de carbone ou d'azote, dans la mesure où ils ne constituent pas un atome de tête de pont, les atomes de carbone sont éventuellement substitués par un atome d'oxygène, un groupe alkyle inférieur, alcoxy inférieur, aryle, aralkyle ou aryloxy, et les atomes d'azote sont éventuellement substitués par un groupe alkyle inférieur, aralkyle ou aryle, et

Y représente une chaîne hydrocarbonée insaturée, comportant 3 - 5 maillons, qui est éventuellement interrompue par des atomes d'azote ou de soufre, ou qui comprend ces hétéroatomes au début ou à la fin de la chaîne, les atomes de carbone étant éventuellement substitués par un groupe alkyle inférieur, alcoxy inférieur, aralkyle, aryloxy ou aryle et/ou les atomes d'azote étant éventuellement substitués par un groupe alkyle inférieur, aralkyle ou aryle, deux restes alkyle inférieurs voisins formant éventuellement un groupe alkylène saturé avec 3 - 5 atomes de carbone,

et au total, la somme des hétéroatomes dans le système bicyclique est au maximum de 3,

et

X représente un anion d'un acide organique ou minéral.

**2.** Composés selon la revendication 1, caractérisés en ce que Z représente

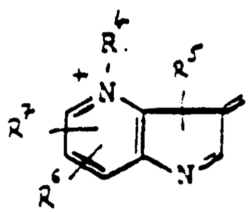

(IV)

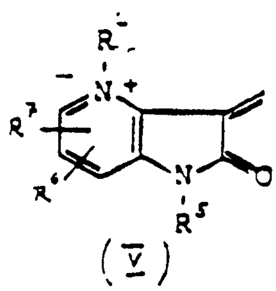

(V)

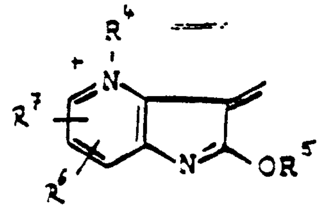

(VI)

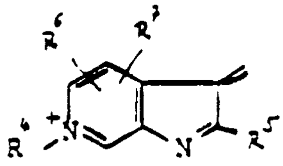

(VII)

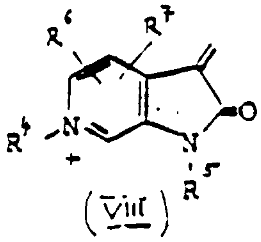

(VIII)

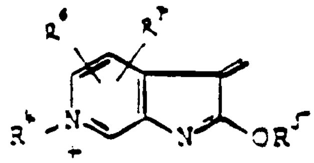

(IX)

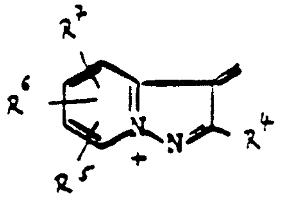

(X)

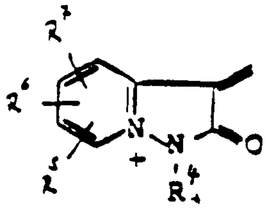

(XI)

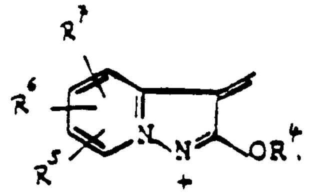

(XII)

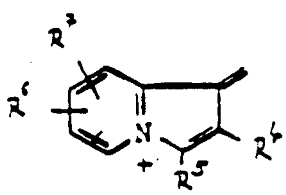

(XIII)

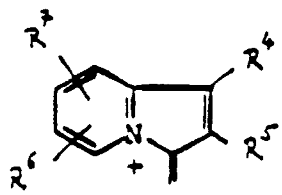

(XIV)

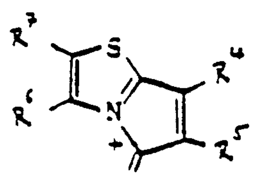

(XV)

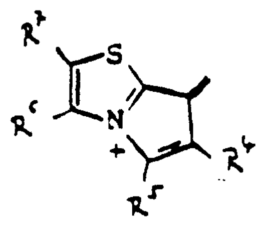

XVI

et

$R^4$ - $R^7$, qui peuvent être identiques ou différents, représentent un atome d'hydrogène, un groupe alkyle inférieur, alcoxy inférieur, aralkyle, aryloxy ou aryle.

3. Procédé de préparation des composés selon la revendication 1, caractérisé en ce que l'on fait réagir des composés de formule générale Ia)

$R^1$-OH (Ia)

dans laquelle
$R^1$ représente un résidu d'aminoacide ou un résidu d'un oligopeptide, dans lequel les groupes amino sont substitués par des groupes protecteurs,
avec
a) des dérivés de phénylènediamine de formule générale XXXIII

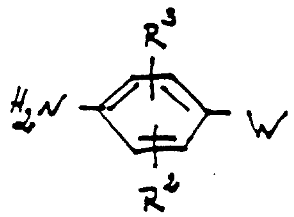

(XXXIII)

dans laquelle
$R^2$ et $R^3$, qui peuvent être identiques ou différents, représentent un atome d'hydrogène, un groupe alkyle inférieur, alcoxy inférieur, carboxy, alcoxy(inférieur)carbonyle ou un groupe carboxamido éventuellement substitué par un groupe alkyle inférieur, ou dans le cas où $R^2$ et $R^3$ sont voisins, un groupe -CH = CH-CH = CH-, et
W représente un groupe amino substitué par un groupe protecteur,
ensuite on sépare le groupe aminoprotecteur du groupe W et l'on oxyde le composé ainsi obtenu avec des groupes $NH_2$ libres, ou
b) on fait réagir des dérivés de p-nitroaniline de formule générale XXXIV

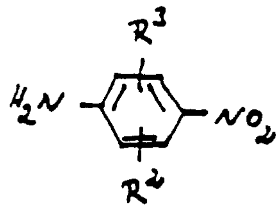

(XXXIV)

dans laquelle
$R^2$, $R^3$ ont les significations mentionnées ci-dessus,
on réduit le composé nitro ainsi obtenu en hydroxylamine correspondante et ensuite, on oxyde les composés nitroso ainsi obtenus, de formule générale XVII

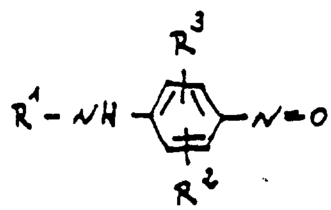

(XVII)

dans laquelle
$R^1$-$R^3$ ont les significations mentionnées ci-dessus, dans des conditions de catalyse acide, avec des composés de formule générale XVIII

Z'-H (XVIII)

dans laquelle
Z' représente un système hétérocyclique, bicyclique, contenant au moins un atome d'azote, non chargé, de formule générale (XIXa) ou (XIXb)

(XIX a) 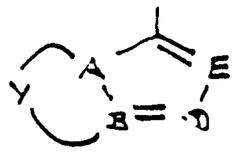 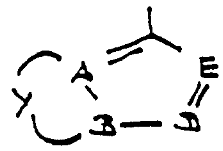 (XIX b)

dans laquelle

A, B, D et E représentent chacun un atome de carbone ou d'azote, dans la mesure où ils ne représentent pas un atome de tête de pont, les atomes de carbone sont éventuellement substitués par un atome d'oxygène, un groupe alkyle inférieur, alcoxy inférieur, aryle, aralkyle ou aryloxy et les atomes d'azote sont éventuellement substitués par un groupe alkyle inférieur, aralkye ou aryle,

Y représente une chaîne hydrocarbonée insaturée comportant 3 - 5 maillons, qui est éventuellement interrompue par des atomes d'azote ou de soufre, ou qui comprend ces hétéroatomes au début ou à la fin de la chaîne,

les atomes de carbone pouvant être éventuellement substitués par un groupe alkyle inférieur, alcoxy inférieur, aralkyle, aryloxy ou aryle et/ou les atomes d'azote pouvant être éventuellement substitués par un groupe alkyle inférieur, aralkyle ou aryle, deux résidus alkyle inférieur voisins formant éventuellement un groupe alkylène saturé comportant 3 - 5 atomes de carbone,

la somme totale des hétéroatomes dans le système bicyclique est de 3 au maximum, les doubles liaisons de la chaîne insaturée Y sont conjuguées par rapport au système de double liaison du cycle à cinq maillons et/ou, lorsque A ou B représente un atome d'azote, celui-ci constitue des structures d'énamine pour les deux systèmes cycliques,

et ensuite, les groupes aminoprotecteurs sont éventuellement éliminés totalement ou en partie.

**4.** Composés de formule générale XVII

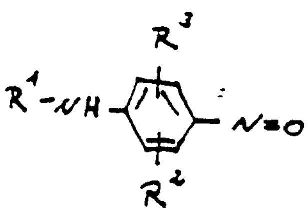 (XVII)

dans laquelle

$R^1$ représente un résidu d'aminoacide ou le résidu d'un oligopeptide, dans lequel les groupes amino sont éventuellement substitués par des groupes protecteurs, et

$R^2$, $R^3$, qui sont identiques ou différents, représentent un atome d'hydrogène, un groupe alkyle inférieur, alcoxy inférieur, carboxy, alcoxy(inférieur)carbonyle ou un groupe carboxamido éventuellement substitué par un groupe alkyle inférieur, ou dans le cas où $R^2$ ou $R^3$ sont voisins, un groupe $-CH=CH-CH=CH-$.

**5.** Procédé de préparation des composés de formule générale XVII

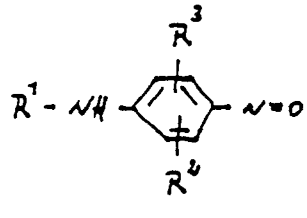

dans laquelle

$R^1$ représente un résidu d'aminoacide ou le résidu d'un oligopeptide, dans lequel les groupes amino sont éventuellement substitués par des groupes protecteurs, et

$R^2$ et $R^3$, qui sont identiques ou différents, représentent un atome d'hydrogène, un groupe alkyle inférieur, alcoxy inférieur, carboxy, alcoxy(inférieur)carbonyle ou un groupe carboxamido éventuellement substitué par un groupe alkyle inférieur, ou dans le cas où $R^2$ et $R^3$ sont voisins, un groupe -CH = CH-CH = CH-,

caractérisé en ce que l'on fait réagir un composé de formule générale I a)

$R^1$-OH (I a)

dans laquelle

$R^1$ représente un résidu d'aminoacide ou le résidu d'un oligopeptide, dans lequel les groupes amino sont substitués par des groupes protecteurs,

avec

a) un dérivé de phénylènediamine de formule générale XXXIII

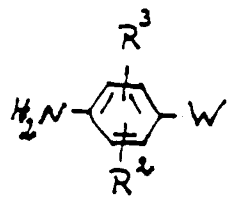

dans laquelle

$R^2$ et $R^3$ ont les significations mentionnées ci-dessus et

W représente un groupe amino substitué par un groupe protecteur, ensuite on sépare le groupe aminoprotecteur du groupe W et l'on oxyde le composé résultant comportant des groupes $NH_2$ libres, ou

b) un dérivé de p-nitroaniline de formule générale XXXIV

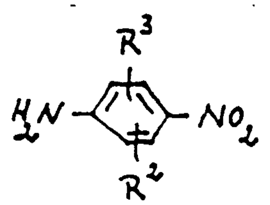

dans laquelle

$R^2$ et $R^3$ ont les significations mentionnées ci-dessus,

on réduit le composé nitro ainsi obtenu en hydroxylamine ou amine correspondante et ensuite, on oxyde.

**6.** Utilisation des composés selon la revendication 4, pour la préparation des composés selon les revendications 1 - 2.

**7.** Utilisation des composés selon les revendications 1 - 2, pour la détermination d'enzymes coupant des liaisons peptidiques.

**8.** Utilisation des composés selon la revendication 7, pour la détermination des facteurs de coagulation.

**9.** Agent de diagnostic pour la détermination d'enzymes coupant des liaisons peptidiques, contenant un ou plusieurs substrats chromogènes, une substance tampon appropriée ainsi qu'éventuellement d'autres adjuvants usuels, caractérisé en ce qu'il contient, comme substrats chromogènes, des composés selon les revendications 1 - 2.